# EUROPEAN PATENT APPLICATION

(11) **EP 1 801 202 A1**
(43) Date of publication of application: **27.06.2007**
(21) Application number: 05781377.6
(22) Date of filing: 02.09.2005
(51) Int. Cl.: C12N 5/06, C12N 15/09

(54) **PROCESS FOR PRODUCING DENDRITIC CELL HAVING ACQUIRED CTL INDUCING CAPABILITY**

(30) Priority: 03.09.2004 JP 2004257776
(71) Applicant: Medical and Biological Laboratories Co., Ltd., Nagoya-shi, Aichi 460-0002 (JP); Keio University, Tokyo 108-8345 (JP)
(72) Inventor: IMAI, Jun, 2010003 (JP); MARUYA, Mikako, 1710031 (JP); HASEGAWA, Hironori, 3960011 (JP); KOYASU, Shigeo, 1770042 (JP); YAHARA, Ichiro, Yokohama-shi, Kanagawa 2220012 (JP)
(74) Representative: Denison, Christopher Marcus
(86) International application number: PCT/JP2005/016111
(87) International publication number: WO 2006/025526

(57) **Abstract**

The present inventors worked on elucidating the mechanism of antigen cross-presentation by dendritic cells, and revealed that foreign antigens taken up in dendritic cells are degraded by proteasomes after undergoing polyubiquitination. Based on the novel finding that polyubiquitination is involved in cross-presentation, the promotion of polyubiquitination was attempted by a number of methods, and the promotion of antigen presentation was confirmed. These methods enable the production of dendritic cells effective for inducing CTL activation.

SEQUENCE LISTING

<110> MEDICAL AND BIOLOGICAL LABORATORIES CO., LTD.
KEIO UNIVERSITY

<120> PROCESS FOR PRODUCING DENDRITIC CELL HAVING ACQUIRED CTL INDUCING CAPABILITY

<130> M3-A0403P

<150> JP 2004-257776
<151> 2004-09-03

<160> 15

<170> PatentIn version 3.1

<210> 1
<211> 733
<212> PRT
<213> Sus scrofa

<400> 1

Met Pro Glu Glu Thr Gln Thr Gln Asp Gln Pro Met Glu Glu Glu Glu
1               5                   10                  15

Val Glu Thr Phe Ala Phe Gln Ala Glu Ile Ala Gln Leu Met Ser Leu
            20                  25                  30

Ile Ile Asn Thr Phe Tyr Ser Asn Lys Glu Ile Phe Leu Arg Glu Leu
        35                  40                  45

Ile Ser Asn Ser Ser Asp Ala Leu Asp Lys Ile Arg Tyr Glu Ser Leu

    50                  55                  60

Thr Asp Pro Ser Lys Leu Asp Ser Gly Lys Glu Leu His Ile Asn Leu
65                  70                  75                  80

Ile Pro Asn Lys Gln Asp Arg Thr Leu Thr Ile Val Asp Thr Gly Ile
                85                  90                  95

Gly Met Thr Lys Ala Asp Leu Ile Asn Asn Leu Gly Thr Ile Ala Lys
            100                 105                 110

Ser Gly Thr Lys Ala Phe Met Glu Ala Leu Gln Ala Gly Ala Asp Ile
        115                 120                 125

Ser Met Ile Gly Gln Phe Gly Val Gly Phe Tyr Ser Ala Tyr Leu Val
    130                 135                 140

Ala Glu Lys Val Thr Val Ile Thr Lys His Asn Asp Asp Glu Gln Tyr
145                 150                 155                 160

Ala Trp Glu Ser Ser Ala Gly Gly Ser Phe Thr Val Arg Thr Asp Thr
                165                 170                 175

Gly Glu Pro Met Gly Arg Gly Thr Lys Val Ile Leu His Leu Lys Glu
            180                 185                 190

Asp Gln Thr Glu Tyr Leu Glu Glu Arg Arg Ile Lys Glu Ile Val Lys

        195                 200                 205

Lys His Ser Gln Phe Ile Gly Tyr Pro Ile Thr Leu Phe Val Glu Lys
    210                 215                 220

Glu Arg Asp Lys Glu Val Ser Asp Asp Glu Ala Glu Glu Lys Glu Asp
225                 230                 235                 240

Lys Glu Glu Glu Lys Glu Lys Glu Glu Lys Glu Ser Glu Asp Lys Pro
                245                 250                 255

Glu Ile Glu Asp Val Gly Ser Asp Glu Glu Glu Glu Glu Lys Lys Asp
            260                 265                 270

Gly Asp Lys Lys Lys Lys Lys Lys Ile Lys Glu Lys Tyr Ile Asp Gln
        275                 280                 285

Glu Glu Leu Asn Lys Thr Lys Pro Ile Trp Thr Arg Asn Pro Asp Asp
    290                 295                 300

Ile Thr Asn Glu Glu Tyr Gly Glu Phe Tyr Lys Ser Leu Thr Asn Asp
305                 310                 315                 320

Trp Glu Asp His Leu Ala Val Lys His Phe Ser Val Glu Gly Gln Leu
                325                 330                 335

Glu Phe Arg Ala Leu Leu Phe Val Pro Arg Arg Ala Pro Phe Asp Leu

            340                 345                 350

Phe Glu Asn Arg Lys Lys Lys Asn Asn Ile Lys Leu Tyr Val Arg Arg
        355                 360                 365

Val Phe Ile Met Asp Asn Cys Glu Glu Leu Ile Pro Glu Tyr Leu Asn
    370                 375                 380

Phe Ile Arg Gly Val Val Asp Ser Glu Asp Leu Pro Leu Asn Ile Ser
385                 390                 395                 400

Arg Glu Met Leu Gln Gln Ser Lys Ile Leu Lys Val Ile Arg Lys Asn
                405                 410                 415

Leu Val Lys Lys Cys Leu Glu Leu Phe Thr Glu Leu Ala Glu Asp Lys
            420                 425                 430

Glu Asn Tyr Lys Lys Phe Tyr Glu Gln Phe Ser Lys Asn Ile Lys Leu
        435                 440                 445

Gly Ile His Glu Asp Ser Gln Asn Arg Lys Lys Leu Ser Glu Leu Leu
    450                 455                 460

Arg Tyr Tyr Thr Ser Ala Ser Gly Asp Glu Met Val Ser Leu Lys Asp
465                 470                 475                 480

Tyr Cys Thr Arg Met Lys Glu Asn Gln Lys His Ile Tyr Tyr Ile Thr

                485                 490                 495

Gly Glu Thr Lys Asp Gln Val Ala Asn Ser Ala Phe Val Glu Arg Leu
            500                 505                 510

Arg Lys His Gly Leu Glu Val Ile Tyr Met Ile Glu Pro Ile Asp Glu
        515                 520                 525

Tyr Cys Val Gln Gln Leu Lys Glu Phe Glu Gly Lys Thr Leu Val Ser
    530                 535                 540

Val Thr Lys Glu Gly Leu Glu Leu Pro Glu Asp Glu Glu Glu Lys Lys
545                 550                 555                 560

Lys Gln Glu Glu Lys Lys Thr Lys Phe Glu Asn Leu Cys Lys Ile Met
                565                 570                 575

Lys Asp Ile Leu Glu Lys Lys Val Glu Lys Val Val Val Ser Asn Arg
            580                 585                 590

Leu Val Thr Ser Pro Cys Cys Ile Val Thr Ser Thr Tyr Gly Trp Thr
        595                 600                 605

Ala Asn Met Glu Arg Ile Met Lys Ala Gln Ala Leu Arg Asp Asn Ser
    610                 615                 620

Thr Met Gly Tyr Met Ala Ala Lys Lys His Leu Glu Ile Asn Pro Asp

625                 630                 635                 640

His Ser Ile Ile Glu Thr Leu Arg Gln Lys Ala Glu Ala Asp Lys Asn
                645                 650                 655

Asp Lys Ser Val Lys Asp Leu Val Ile Leu Leu Tyr Glu Thr Ala Leu
            660                 665                 670

Leu Ser Ser Gly Phe Ser Leu Glu Asp Pro Gln Thr His Ala Asn Arg
        675                 680                 685

Ile Tyr Arg Met Ile Lys Leu Gly Leu Gly Ile Asp Glu Asp Asp Pro
    690                 695                 700

Thr Ala Asp Asp Ser Ser Ala Ala Val Thr Glu Glu Met Pro Pro Leu
705                 710                 715                 720

Glu Gly Asp Asp Asp Thr Ser Arg Met Glu Glu Val Asp
                725                 730

<210>  2
<211>  2456
<212>  DNA
<213>  Sus scrofa

<400>  2
agccggcgct gtcgttcagc ctagatgccc gaggaaaccc agacccaaga ccagccgatg      60

gaggaggagg aggtggagac gttcgccttc caggcggaaa tcgcccagtt gatgtcgttg     120

atcatcaaca ctttctactc gaacaaggag atctttctga gggagctcat ttccaactcg 180

tccgatgctt tggacaagat cagatacgaa agcctgacgg atcccagtaa actagactcc 240

gggaaagagc tgcacattaa tctcattccg aacaagcaag accggaccct cacgatagtg 300

gacaccggca tcggcatgac caaggccgac ttgatcaata accttggtac gatcgccaag 360

tctgggacca aggcgttcat ggaggctttg caggccggtg ccgatatctc gatgattggc 420

cagttcggtg tcggcttcta ctctgcgtac ctggtcgctg agaaagtgac cgttatcacc 480

aaacacaacg atgacgagca gtatgcctgg gagtcttctg caggaggatc tttcaccgtt 540

aggacagaca caggagaacc tatgggtcgt ggaacaaagg ttattctaca tctgaaagaa 600

gaccaaactg agtacttgga agaaaggaga ataaaggaga ttgtgaagaa acactctcag 660

tttattggct accccattac tctcttcgtg gagaaggaac gtgataaaga agtcagtgac 720

gacgaggcgg aagaaaagga agacaaagag gaagaaaagg agaaagaaga gaaggaatct 780

gaggataaac cggagataga agatgttggt tctgatgaag aggaagaaga aaagaaggat 840

ggtgacaaga agaagaagaa gaagatcaag gagaagtata ttgatcaaga ggaactcaac 900

aagacaaagc ctatctggac cagaaacccc gatgacatca ctaatgaaga gtacggggag 960

ttctataaga gcttgaccaa tgactgggag gatcacttgg ctgtgaagca cttttcagtc 1020

gaagggcagt tggagttcag agcccttctt ttcgtcccaa gacgcgctcc tttcgactta 1080

tttgaaaaca gaaagaagaa gaacaacatc aagctgtatg ttcgcagagt gttcatcatg 1140

gacaactgcg aggagctcat ccctgagtat ctgaatttca ttagaggcgt ggtggactct 1200

gaggatcttc ctctgaacat ttctcgtgag atgttgcaac aaagcaaaat tttgaaagtc 1260

atcaggaaga atctggtcaa gaaatgcttg gagctcttta ctgaattggc cgaagataaa 1320

gagaattaca agaagttcta tgagcagttc tctaaaaata ttaagcttgg aatacatgaa 1380

gattctcaaa atcggaagaa gctttccgag ctgttgaggt actacacttc tgcttctggc 1440

gacgagatgg tttccctcaa ggactattgc accagaatga aggagaacca gaaacacatc 1500

tattacatca caggtgagac caaggaccag gtggccaact cggccttcgt ggaacgtctg 1560

cggaagcacg gcctggaggt gatctacatg atcgagccca tcgatgagta ctgtgtgcag 1620

cagctgaagg agtttgaggg gaagacctta gtgtcagtca ccaaagaggg cctggagctc 1680

ccggaagatg aagaggagaa gaagaaacag gaggagaaga agacaaagtt tgaaaacctc 1740

tgcaagatca tgaaggacat cttggagaag aaagtcgaaa aggtggttgt gtcgaaccgg 1800

ttggtgacct caccgtgctg cattgtcaca agcacatacg gctggacagc aaacatggag 1860

agaatcatga aggctcaagc cctgagagac aactcgacaa tgggttacat ggcagcaaag 1920

aagcacctgg agataaatcc agaccattcc atcatcgaga ccctgaggca aaaggcagag 1980

gcggacaaga acgacaagtc cgtgaaggat ctggtcatcc tgctgtacga aaccgctctg 2040

ctgtcttctg gcttcagcct ggaagatccc cagacgcacg ccaacaggat ctacaggatg 2100

atcaaacttg gtcttggtat tgatgaggac gaccccaccg ccgacgacag cagcgctgct 2160

gtgacggagg agatgccacc cctggaaggg gacgacgaca cgtcccgcat ggaggaagtc 2220

gattaggctc cgcctgaggc ttgcttgtcc cctgttaaaa cgctgtctgc attccctctg 2280

acgatatatt ttcaagtatt ttatttttgt taacatttaa aggttgtatg gcatgacagt 2340

tccttaaggg aaaataagat tcctttgtac taagtgtgac actgtgacat ttgagcacta 2400

aagccgagct tgtcctttcc tagttccagt tggcttcttt aacagacggc cctccc 2456

<210> 3
<211> 4
<212> PRT
<213> Artificial

<220>
<223> an artificially synthesized peptide sequence

<400> 3

Lys Asp Glu Leu
1

<210> 4
<211> 12
<212> DNA
<213> Artificial

<220>
<223> an artificially synthesized nucleotide sequence

<400> 4
aaagatgaat ta 12

<210> 5
<211> 4

<212>  PRT
<213>  Artificial

<220>
<223>  an artificially synthesized peptide sequence

<400>  5

His Asp Glu Leu
1

<210>  6
<211>  6
<212>  PRT
<213>  Artificial

<220>
<223>  an artificially synthesized peptide sequence

<400>  6

Asp Glu Lys Lys Met Pro
1               5

<210>  7
<211>  19
<212>  DNA
<213>  Artificial

<220>
<223>  an artificially synthesized oligonucleotide for siRNA

<400>  7
aatgatcatt actatcggt                                                    19

<210> 8
<211> 21
<212> DNA
<213> Artificial

<220>
<223> an artificially synthesized oligonucleotide for siRNA

<400> 8
aatccttgaa tgaagtaggc t 21

<210> 9
<211> 19
<212> DNA
<213> Artificial

<220>
<223> an artificially synthesized oligonucleotide for siRNA

<400> 9
cagtatcgag gaacggcgc 19

<210> 10
<211> 24
<212> DNA
<213> Artificial

<220>
<223> an artificially synthesized primer sequence

<400> 10
gcgggatcca tgggctccat cggt 24

<210> 11
<211> 33
<212> DNA
<213> Artificial

<220>
<223> an artificially synthesized primer sequence

<400> 11
cgcggatcct agctagcagg ggaaacacat ctg 33

<210> 12
<211> 95
<212> DNA
<213> Artificial

<220>
<223> an artificially synthesized nucleotide sequence

<400> 12
gcgactagtg ctgaagatta tgaatatcct tctagatcta atggtaccaa aaataatact 60

tctcaagcta gcaaagatga attataggat cccgc 95

<210> 13
<211> 95
<212> DNA
<213> Artificial

<220>
<223> an artificially synthesized nucleotide sequence

<400>  13

gcgggatcct ataattcatc tttgctagct tgagaagtat tatttttggt accattagat      60

ctagaaggat attcataatc ttcagcacta gtcgc                                 95

<210>  14
<211>  413
<212>  PRT
<213>  Artificial

<220>
<223>  an artificially synthesized peptide sequence

<400>  14

Met Gly Ser Ile Gly Ala Ala Ser Met Glu Phe Cys Phe Asp Val Phe
1               5                   10                  15

Lys Glu Leu Lys Val His His Ala Asn Glu Asn Ile Phe Tyr Cys Pro
            20                  25                  30

Ile Ala Ile Met Ser Ala Leu Ala Met Val Tyr Leu Gly Ala Lys Asp
        35                  40                  45

Ser Thr Arg Thr Gln Ile Asn Lys Val Val Arg Phe Asp Lys Leu Pro
    50                  55                  60

Gly Phe Gly Asp Ser Ile Glu Ala Gln Cys Gly Thr Ser Val Asn Val
65                  70                  75                  80

His Ser Ser Leu Arg Asp Ile Leu Asn Gln Ile Thr Lys Pro Asn Asp
                85                  90                  95

Val Tyr Ser Phe Ser Leu Ala Ser Arg Leu Tyr Ala Glu Glu Arg Tyr
            100                 105                 110

Pro Ile Leu Pro Glu Tyr Leu Gln Cys Val Lys Glu Leu Tyr Arg Gly
        115                 120                 125

Gly Leu Glu Pro Ile Asn Phe Gln Thr Ala Ala Asp Gln Ala Arg Glu
    130                 135                 140

Leu Ile Asn Ser Trp Val Glu Ser Gln Thr Asn Gly Ile Ile Arg Asn
145                 150                 155                 160

Val Leu Gln Pro Ser Ser Val Asp Ser Gln Thr Ala Met Val Leu Val
                165                 170                 175

Asn Ala Ile Val Phe Lys Gly Leu Trp Glu Lys Ala Phe Lys Asp Glu
            180                 185                 190

Asp Thr Gln Ala Met Pro Phe Arg Val Thr Glu Gln Glu Ser Lys Pro
        195                 200                 205

Val Gln Met Met Tyr Gln Ile Gly Leu Phe Arg Val Ala Ser Met Ala
    210                 215                 220

Ser Glu Lys Met Lys Ile Leu Glu Leu Pro Phe Ala Ser Gly Thr Met
225                 230                 235                 240
Ser Met Leu Val Leu Leu Pro Asp Glu Val Ser Gly Leu Glu Gln Leu
                245                 250                 255
Glu Ser Ile Ile Asn Phe Glu Lys Leu Thr Glu Trp Thr Ser Ser Asn
            260                 265                 270
Val Met Glu Glu Arg Lys Ile Lys Val Tyr Leu Pro Arg Met Lys Met
        275                 280                 285
Glu Glu Lys Tyr Asn Leu Thr Ser Val Leu Met Ala Met Gly Ile Thr
    290                 295                 300
Asp Val Phe Ser Ser Ser Ala Asn Leu Ser Gly Ile Ser Ser Ala Glu
305                 310                 315                 320
Ser Leu Lys Ile Ser Gln Ala Val His Ala Ala His Ala Glu Ile Asn
                325                 330                 335
Glu Ala Gly Arg Glu Val Val Gly Ser Ala Glu Ala Gly Val Asp Ala
            340                 345                 350
Ala Ser Val Ser Glu Glu Phe Arg Ala Asp His Pro Phe Leu Phe Cys
        355                 360                 365

Ile Lys His Ile Ala Thr Asn Ala Val Leu Phe Phe Gly Arg Cys Val
    370                 375                 380

Ser Pro Ala Ser Ala Glu Asp Tyr Glu Tyr Pro Ser Arg Ser Asn Gly
385                 390                 395                 400

Thr Lys Asn Asn Thr Ser Gln Ala Ser Lys Asp Glu Leu
                405                 410

<210> 15
<211> 8
<212> PRT
<213> Gallus gallus

<400> 15

Ser Ile Ile Asn Phe Glu Lys Leu

## Description

### Technical Field

The present invention relates to methods for producing dendritic cells that have acquired CTL-inducing ability, wherein the methods are based on cross-presentation mechanisms by dendritic cells.

### Background Art

When cells in a living body undergo cancerization, normally, immune defense mechanisms function to remove the cancerized cells. Cancer is thought to develop when the cancerized cells escape the surveillance of the immune defense mechanisms. The cancerized cells are mainly removed through cellular immunity. If a cellular immune mechanism is activated by a suitable procedure, it can act to strongly attack and remove cancers that have formed. Activation of cellular immunity may become a method for efficient removal of cancer cells; however, activation of cellular immunity cannot be separated from induction of immunological tolerance. Therefore, how to achieve an activation that is efficient and specific to the target is a major issue in tumor immunity.

Cellular immunity is a type of acquired immunity. Acquired immunity is classified into two types: humoral immunity and cellular immunity. Humoral immunity is an immune reaction mediated by antibodies against infection by bacteria, parasites, and such, during which B cells, which produce antibodies, are mainly activated. On the other hand, cellular immunity is a reaction that directly destroys cancerized cells and virus-infected cells, during which CD8⁺ cytotoxic T cells are activated.

Cytotoxic T cells (cytotoxic T lymphocytes; CTLs) are cells that specifically attack target cells such as virus-infected cells or cancer cells, and play a central role in cellular immunity. CTLs induce target cell apoptosis by releasing cytolytic granules including perforin, granzyme and such, or through interactions between FasL on the surfaces of CTLs and Fas on target cell surfaces. In order for CTLs to exhibit such cytotoxicity, CD8⁺ T cells must be activated.

T cells use T cell receptors (TcRs) expressed on their cell membrane to recognize a group of protein complexes called major histocompatibility complexes (MHCs) on other cells. MHC molecules encoded by MHC genes form complexes with oligopeptides produced by the degradation of proteins in cells, and are then expressed on cell surfaces.

There are two types of MHC molecules: Class I and Class II. MHC II molecules comprise an α chain and a β chain, are only expressed in antigen-presenting cells, mainly present peptides derived from antigens taken up from outside the cell (infectious pathogens and such) to helper T cells, and play an essential role in the expression of humoral immunity.

In contrast, MHC I molecules, which comprise a main chain and subchain (β2 microglobulin), are expressed on the cell membrane of almost all nucleated cells. Generally, MHC I molecules are bound to oligopeptides derived from the degradation products of proteins in their own cell, and are self markers in cellular immunity. With regards to discriminating between self and non-self, MHC Class I molecules presenting self-protein-derived peptides are recognized as self, and a cellular immune reaction is not induced. On the other hand, when MHC Class I molecules are presenting peptides derived from non-self proteins (for example, cancerized cells, virus-infected cells, transplanted organs, or such), a cellular immune reaction is induced.

Activation of cellular immunity requires the presentation on an MHC I molecule of a non-self-derived peptide along with a costimulatory factor to CD8 antigen-positive T cells. If this activation does not occur efficiently, CD8⁺ T cells that recognize non-self can not differentiate into activated cells (CTLs) exhibiting cytotoxicity against cells presenting non-self antigens, even though they may be present.

Dendritic cells, along with macrophages and B cells, are referred to as professional antigen-presenting cells, and play a notably important and special role in the presentation of foreign antigens. Dendritic cells take up carcasses or fragments of cancerized cells or virus-infected cells and present proteins included in these on MHC class I molecules. Therefore, in macrophages and B cells, foreign antigens are presented on MHC class II molecules, whereas in dendritic cells, foreign antigens are presented on MHC class I as well as MHC class II molecules. This phenomenon is referred to as antigen cross-presentation and is characteristic of dendritic cells. Cross-presentation of foreign antigens by dendritic cells plays a critically important role in the removal of infected cells and cancer cells.

Foreign antigenic proteins taken up by dendritic cells are transported and degraded, then peptides derived from the antigenic proteins form complexes with MHC class I molecules, and these are expressed on cell membranes; however, this cellular mechanism is only partly elucidated. So far, the cellular mechanism of cross-presentation by dendritic cells is described as follows: first, antigenic proteins derived from cancer cells or virus-infected cells are taken up into the lumen of dendritic cells (a compartment surrounded by a membrane) by endocytosis (which also comprises phagocytosis, uptake of immune complexes, and such). Antigenic proteins that have been taken up, or their degradation products, translocate to the cytoplasm by passing through the membrane *via* an unknown mechanism. They are then degraded by proteasomes, in the same way as for endogenous antigenic proteins. Peptides which are the degradation products are transported to the ER by TAP transporters, then form complexes with MHC I molecules in the ER, and are expressed on the cell membrane *via* an ordinary pathway.

The development of cancer as a disease means the survival of cancer cells that normally would be removed by attack from CTLs activated *via* the above process. In other words, in individuals who have developed cancer, CTLs are thought to remain in their inactive (dormant) state. Therefore, cancer treatment by activating these inactive CTLs (or CD8⁺ T cells) is thought possible. In fact, methods for treating cancer using CTL induction are being studied based on various ideas. For example, currently known techniques use various Hsp fusion proteins as antigens (Non-Patent Documents 1-2), use fusion proteins formed with membrane-spanning bacterial toxin proteins as antigens (Non-Patent Documents 3-9), use ubiquitin fusion proteins as antigens (Non-Patent Documents 10-11), increase CTL inducibility by altering epitopes (Non-Patent Document 12), and modify antigens so that they will bind to scavenger receptors (Non-Patent Document 13). However, truly effective therapeutic methods have not yet been developed.
[Non-Patent Document 1] Proc. Natl. Acad. Sci. USA, Vol.94, pp.13146-13151, November 1997.
[Non-Patent Document 2] International Immunology, Vol.13, No.10, pp.1233-1242.
[Non-Patent Document 3] Traffic 2002; 3: 697-704.
[Non-Patent Document 4] Journal of Virology, Aug. 2001, p.7330-7338.
[Non-Patent Document 5] PNAS July 5, 2000, Vol.97, No.14, 8027-8032.
[Non-Patent Document 6] FEBS Letters 453 (1999)95-99.
[Non-Patent Document 7] The Journal of Immunology, 2000, 165:3301-3308.
[Non-Patent Document 8] Cellular Immunology 203,75-83(2000).
[Non-Patent Document 9] Infection and Immunity, June 2002, p.3249-3258.
[Non-Patent Document 10] The Journal of Biological Chemistry Vol.277, No.41, Issue of October 11, pp.38818-38826, 2002.
[Non-Patent Document 11] Science, 28 May 2004; 304(5675), p.1318-21.
[Non-Patent Document 12] Science, 28 May 2004; 304(5675), p.1314-7.
[Non-Patent Document 13] Immunology, 2004, 112, 211-218.

### Disclosure of the Invention

### Problems to be Solved by the Invention

The present invention was achieved in view of the above circumstances. An objective of the present invention is to elucidate the mechanism of antigen cross-presentation by dendritic cells, and to provide methods for producing dendritic cells effective at inducing CTL activation.

### Means to Solve the Problems

To solve the above problems, the present inventors worked on elucidating the mechanism of antigen cross-presentation by dendritic cells. There are two reactions key to elucidating the mechanism of antigen cross-presentation by dendritic cells: 1) the kind of intracellular compartments the antigenic proteins are taken into from the outside; and 2) the way in which the antigenic proteins taken up or their degradation products pass through the membrane to translocate to the cytoplasm.

To elucidate the above mechanism, cell fractionation and Western blotting were carried out, and the intracellular localization of the foreign antigens taken up as well as the changes taking place over time were observed. These results revealed for the first time that the foreign antigens taken up accumulate in membranous organelles in their undegraded form, and are then degraded *via* the ubiquitin-proteasome pathway. Furthermore, the present inventors realized that the above pathway is essentially the same as ER-associated degradation (ERAD), and hypothesized that that the foreign antigens accumulated in the dendritic cells are degraded by ERAD or by a very similar proteolytic mechanism.

The present inventors set out to prove this hypothesis using three methods. In the first method for proving the hypothesis, coimmunoprecipitation was performed to check for any interaction between the accumulated foreign antigens and the various ER-associated proteins. In the second method, the effect of ERAD-inhibiting treatments on the degradation of foreign antigens was investigated. Thirdly, RNAi methods were used to knock-down an ER-associated protein and the effect on the accumulated foreign antigens was examined.

The results of the above examinations revealed that in dendritic cell cross-presentation, foreign antigens are taken up into membrane vesicles, bind to endoplasmic reticulum molecular chaperones such as BiP and calreticulin, and undergo retrograde transport into the cytoplasm; that foreign antigens that transferred to the cytoplasm are ubiquitinated in their undegraded form; that the ubiquitin E3 ligase involved in the above ubiquitination is CHIP; and that the ubiquitinated foreign antigens are degraded by proteasomes. These are all novel findings discovered for the first time by the present inventors, and all support the above hypothesis.

Based on the novel finding that polyubiquitination is involved in cross-presentation, the present inventors also set out to promote polyubiquitination using various methods. When the method of co-immunizing antigenic proteins and Hsp90 *ex vivo,* and the method of adding a KDEL sequence (SEQ ID NO: 3) to antigenic proteins were carried out, promotion of antigenic protein polyubiquitination and an increase in antigen presentation by dendritic cells were confirmed. According to these results, the present inventors have logically and specifically discovered methods for promoting antigen presentation by dendritic cells through the promotion of polyubiquitination. These methods enable the production of dendritic cells that are effective in inducing CTL activation.

Therefore, the present invention relates to methods for producing dendritic cells that have acquired CTL-inducing ability. Specifically, it provides the following inventions:
(1) a method for producing a dendritic cell that has acquired CTL-inducing ability, wherein the method comprises the step of a polyubiquitination promotion treatment that promotes the polyubiquitination of an antigenic protein in a dendritic cell;
(2) a method for producing a dendritic cell that has acquired CTL-inducing ability, wherein the method comprises the steps of:
   (a) isolating a mammal-derived dendritic cell; and
   (b) a polyubiquitination promotion treatment that promotes the polyubiquitination of an antigenic protein in the dendritic cell;
(3) a method for producing a dendritic cell that has acquired CTL-inducing ability, wherein the method comprises the steps of:
   (a) isolating a mammal-derived dendritic cell;
   (b) a polyubiquitination promotion treatment that promotes the polyubiquitination of an antigenic protein in the dendritic cell; and
   (c) confirming the promotion of polyubiquitination of an antigenic protein in the dendritic cell;
(4) the method of any one of (1) to (3) for producing a dendritic cell that has acquired CTL-inducing ability, wherein the step of a polyubiquitination promotion treatment is a step that comprises contacting an antigenic protein and an HSP with an isolated dendritic cell;
(5) the production method of (4), wherein the HSP is an HSP belonging to the HSP90 family or HSP70 family;
(6) the production method of (5), wherein the HSP belonging to the HSP90 family is HSP90;
(7) the method of any one of (1) to (3) for producing a dendritic cell that has acquired CTL-inducing ability, wherein the step of a polyubiquitination promotion treatment is a step that comprises contacting an antigenic protein, in which an endoplasmic reticulum localization sequence has been added to its carboxyl terminus, with an isolated dendritic cell;
(8) the production method of (7), wherein the endoplasmic reticulum localization sequence is KDEL (SEQ ID NO: 3);
(9) an agent for promoting the polyubiquitination of an antigenic protein, which comprises an HSP as an active ingredient;
(10) the agent of (9) for promoting the polyubiquitination of an antigenic protein, in which the HSP is HSP90;
(11) an agent for promoting the polyubiquitination of an antigenic protein, which comprises a DNA encoding an endoplasmic reticulum localization sequence;
(12) an agent for promoting the polyubiquitination of an antigenic protein, which comprises a vector harboring a DNA encoding an endoplasmic reticulum localization sequence; and
(13) an agent for enhancing the CTL-inducing ability of a dendritic cell, which comprises the agent of any one of (9) to (12) for promoting the polyubiquitination of an antigenic protein, as an active ingredient.

### Brief Description of the Drawings

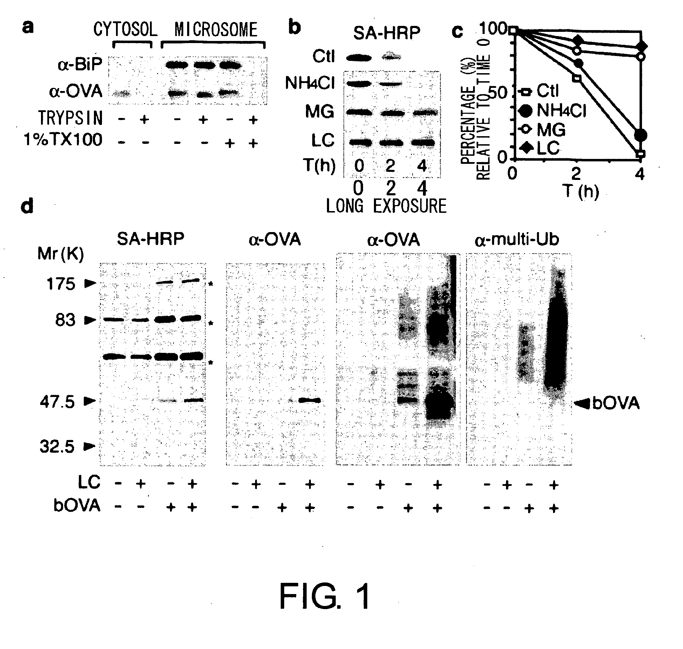

Fig. 1 shows the characteristics of OVA taken up by DC2.4 cells. (a) shows a Western blot of cytosol and microsomal fractions using various antibodies. Trypsin treatment of microsomal fractions did not affect accumulated OVA and BiP. Triton X-100 was used to lyse membranes. (b) shows the effect of proteasome inhibitors and lysosome inhibitors on bOVA accumulation. The proteasome inhibitors MG-132 (MG: 10 µM) and lactastatin (LC: 2 µM) reduced the degradation of bOVA taken up into cells, but NH₄Cl (50 mM) did not reduce this degradation. DC2.4 cells were pulsed with bOVA and chased. bOVA was recovered using SA beads, then separated by SDS-PAGE followed by Western blotting using SA-HRP. Ctl: a control not containing inhibitors. (c) shows the results of quantifying that shown in (b). (d) shows that bOVA is polyubiquitinated before degradation. Cell-bound bOVA has the same molecular weight as intact OVA. Portions of bOVA were polyubiquitinated. Asterisks in the left panel indicate non-specific binding with SA-HRP.

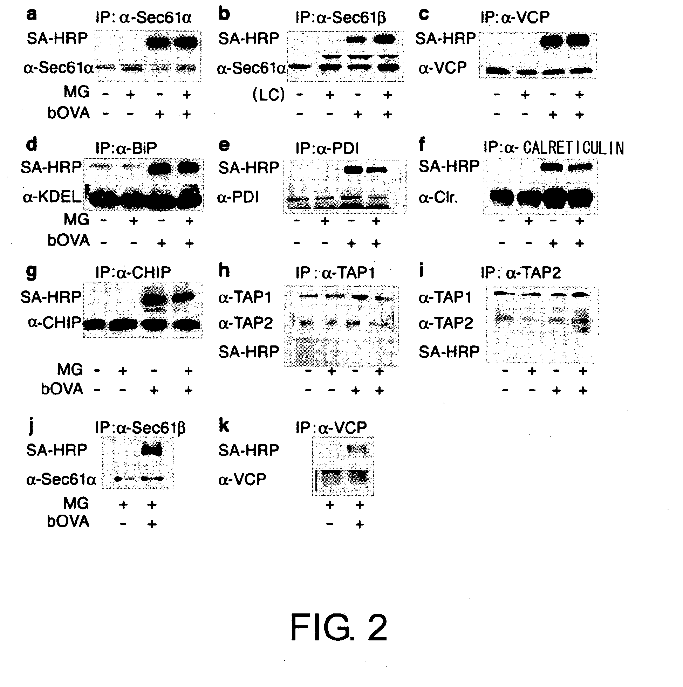

Fig. 2 shows that ERAD-related proteins interact with cell-bound OVA. (a) shows that Sec61α interacts with bOVA. DC2.4 cells were incubated with bOVA and subjected to co-immunoprecipitation with anti-Sec61α. bOVA was added at 2.5 mg mL⁻¹ to cell lysates before immunoprecipitation (the two left-hand lanes in each panel). After SDS-PAGE, blotting was performed using SA-HRP and anti-Sec61α antibody. (b) shows that Sec61β interacts with bOVA. (c) shows that VCP interacts with bOVA. (d) shows that BiP interacts with bOVA. (e) shows that PDI interacts with bOVA. (f) shows that calreticulin interacts with bOVA. (g) shows that CHIP interacts with bOVA. (h) shows that TAP1 does not interact with bOVA. (i) shows that TAP2 does not interact with bOVA. (j) shows that bOVA interacts with Sec61β even in BMDCs. (k) shows that bOVA interacts with VCP even in BMDCs.

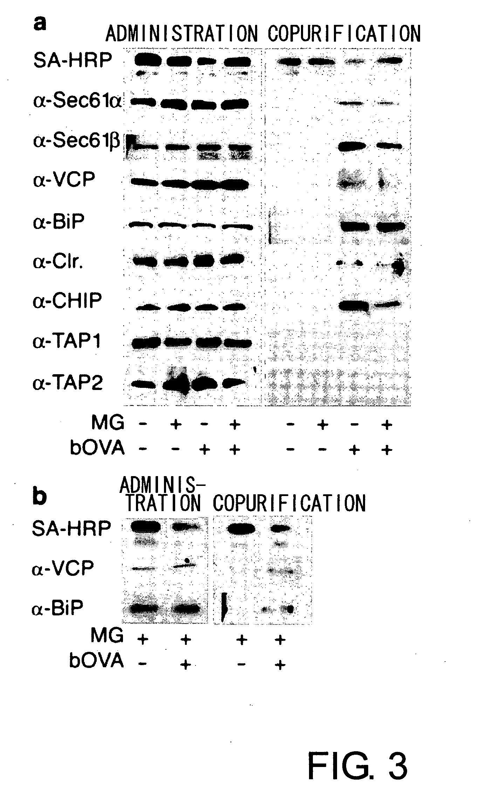

Fig. 3 shows that ERAD-related proteins are co-purified with bOVA taken up in cells. (a) shows co-purification with ERAD-related substances derived from DC2.4 cells. (b) is a figure showing the results of co-purification of VCP and BiP derived from BMDC cells.

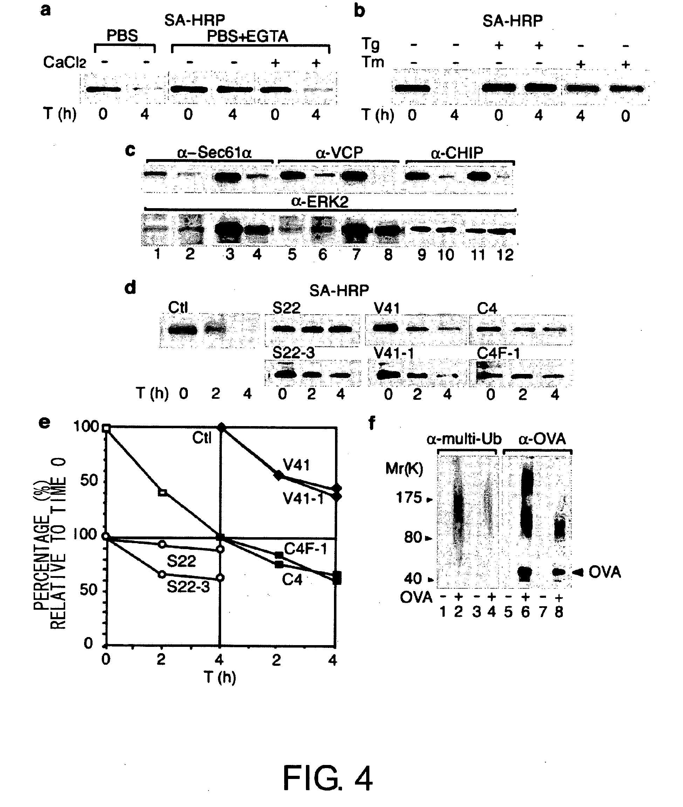

Fig. 4 shows that ERAD inhibition and knock-down of ERAD-related proteins by RNAi methods reduces bOVA degradation. (a) shows that bOVA degradation is inhibited by Ca²⁺ depletion, and recovers upon re-addition of Ca²⁺ (1 mM). (b) shows that bOVA degradation is inhibited by thapsigargin (Tg: 300 nM) or tunicamycin (Tm: 2µg). (c) shows the effect of knocking-down ERAD-related proteins using RNAi methods. Uncloned stable transfectants (S22: Sec61-22; V41: VCP-41; C4: CHIP-4) and cloned knocked-down cell lines (S22-3: derived from S22; V41-1: derived from V41; and C4F-1: derived from C4) into which RNAi was transfected showed reduced expression of ERAD-related proteins (Lanes 1, 3, 5, 7, 9, 10, and 11). The controls are lane 2: S22; lane 4: S22-3; lane 6: V41; lane 8: V41-1; lane 10: C4; and lane 12: C4F-1. (d) shows bOVA degradation when parental DC2.4 cells and knocked-down DC2.4 cells were separately incubated with bOVA. (e) is a diagram quantifying the results of (d) above. (f) shows that polyubiquitination of bOVA was reduced in C4. Lanes 1,2,5, and 6 are the controls, and lanes 3, 4, 7, and 8 are C4.

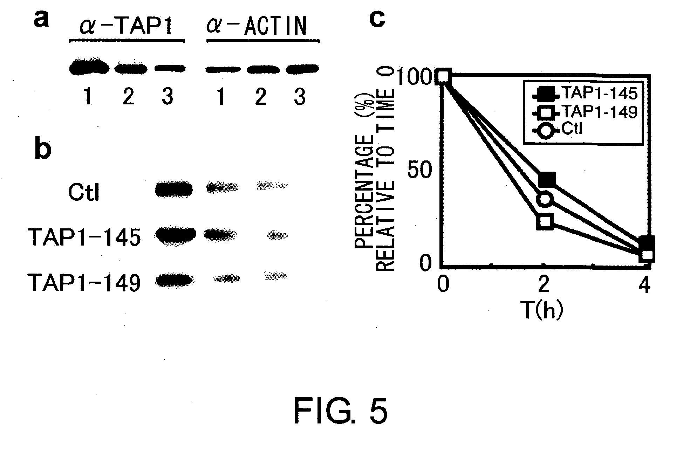

Fig. 5 shows that TAP1 knock-down by RNAi methods does not affect bOVA degradation. (a) Compared to parental DC2.4 cells (lane 1), the uncloned stable transfectants (lane 2: TAP1-145; lane 3: TAP1-149) showed a specific reduction in TAP-1 protein expression. (b) shows the results of measuring bOVA degradation after separately incubating parental DC2.4 cells and knocked-down DC2.4 cells with bOVA. From the left: zero hour, two hours, and four hours. (c) is a diagram quantifying the results of (b) above.

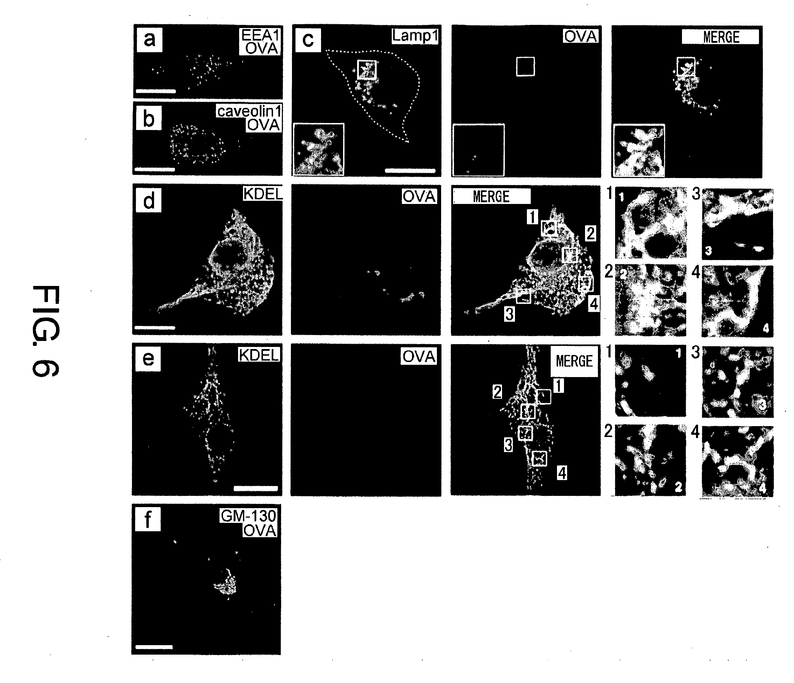

Fig. 6 shows the intracellular localization of exogenously added OVA in DC2.4 cells. The intracellular localization of OVA was detected by labeling with an anti-OVA antibody (red). Early endosomes (a), caveosomes (b), late endosomes (c), ER (d and e) and Golgi apparatus (f) are shown in green. In (c), the cell periphery is indicated by a dotted line. Insets in (c) and (d) show the area of the cell indicated by squares in a higher magnification.

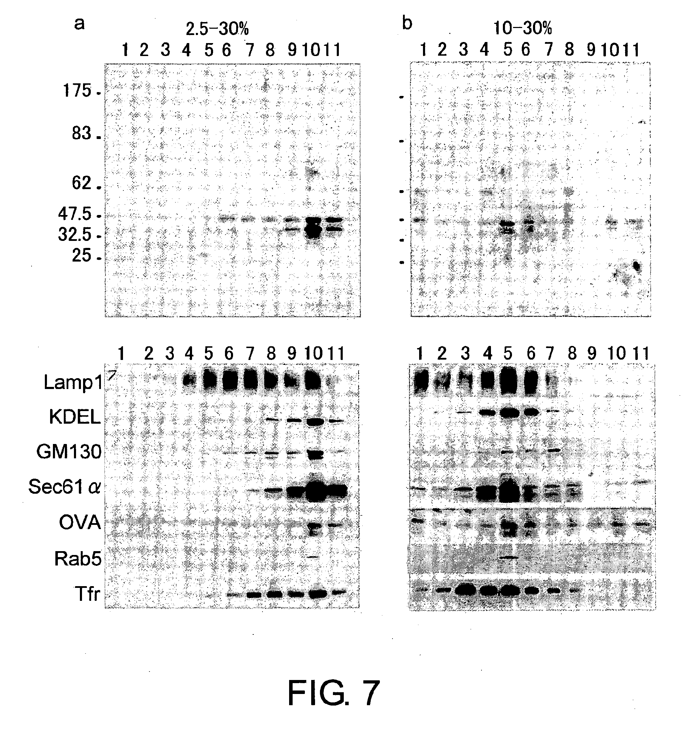

Fig. 7 shows the results of performing density gradient centrifugation on membrane fractions of DC2.4 cells. (a) DC2.4 cells incubated with OVA were homogenized, membrane fractions were prepared and subjected to 2.5-30% Optiprep density gradient centrifugation. The upper panel was obtained by separating each of the fractions by SDS-PAGE and then blotting using an anti-OVA antibody. The lower panel was obtained by blotting the same membrane sheet shown in the upper panel with various antibodies. (b) Membrane fractions were analyzed on a 10-30% density gradient. The lane numbers indicate the fractions of each density gradient.

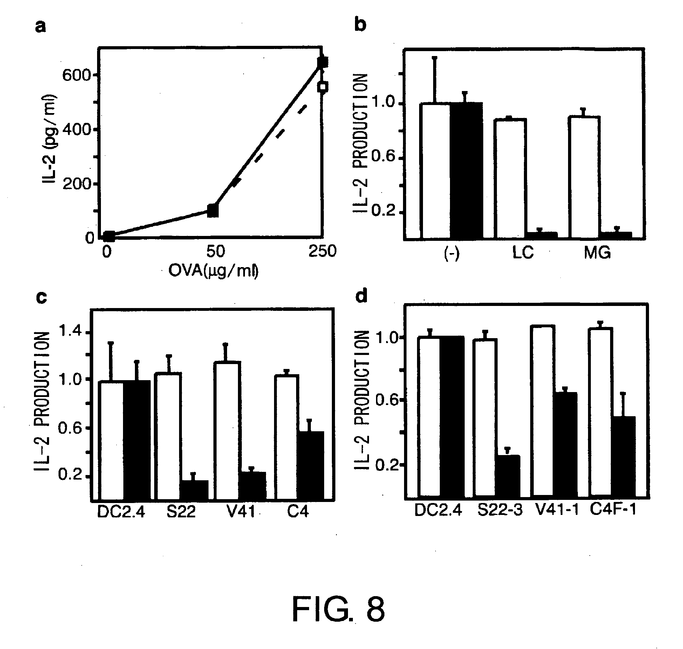

Fig. 8 shows the results of knocking down Sec61, VCP, and CHIP. Parental DC2.4 cells or knocked-down DC2.4 cells were separately incubated with SIINFEKL peptide or OVA, and the stimulation of OT-I CD8⁺ T cells was observed using the IL-2 production level as an index. (a) is a dose-response curve showing the relationship between OVA and IL-2 production. White columns indicate OVA and black columns indicate bOVA. (b) Lactastatin (10 µM) and MG-132 (25 µM) suppressed stimulation by OVA (black bars) but did not suppress stimulation by the SIINFEKL peptide (white bars). (c) A reduction in OVA stimulation was observed with DC2.4 cells in which Sec61 (S22), VCP (V41), or CHIP (C4) was knocked down. (d) Clones from knocked-down cells essentially showed the same results as in (c).

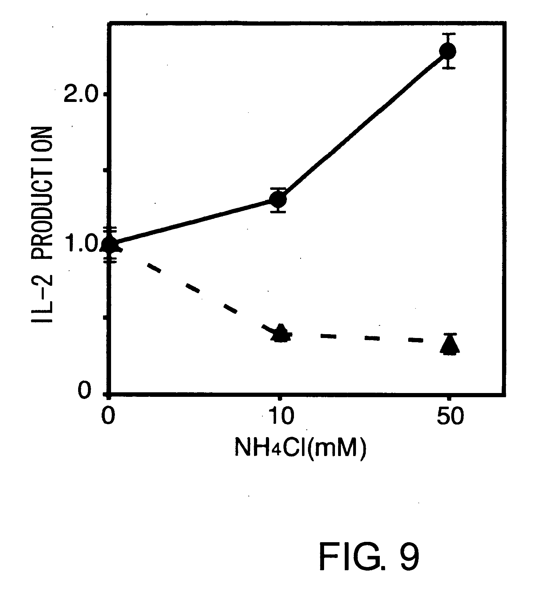

Fig. 9 shows the results of observing the effect of NH₄Cl on antigen presentation by MHC class I and class II molecules on DC2.4 cells. DC2.4 cells pretreated in the presence of NH₄Cl were cultured with OVA, and then the stimulation of OT-I CD8⁺ T cells in response to MHC class I antigen presentation (circles) and stimulation of OT-II CD4⁺ T cells in response to MHC class II antigen presentation (triangles) was determined by measuring IL-2 production levels. IL-2 production level in the presence of NH₄Cl was expressed as a ratio relative to the control (IL-2 production was 700 pg/mL relative to OT-I, and 20 pg/mL relative to OT-II).

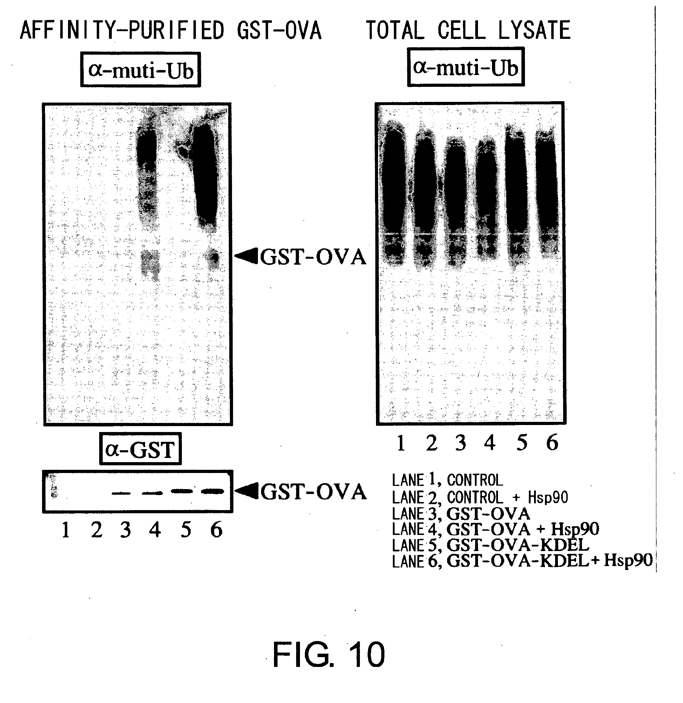

Fig. 10 shows the result of incubating GST-OVA or GST-OVA-KDEL with DC2.4 cells in the presence or absence of HSP90, collecting GST-OVA from the cells, and then performing SDS-PAGE and Western blotting. Lanes 1 and 2 show the results from the cells alone, and lanes 3 and 4 show the results of incubating the cells with GST-OVA in the presence or absence of HSP90. Regarding lane 4 and its control (lane 3), a comparison of the amount of GST-OVA taken up (lower photograph) and the amount of polyubiquitinated GST-OVA shows that the increase in the amount taken up in the presence of HSP90 is not great, but that the increase in polyubiquitination in the presence of HSP90 is very pronounced. When lane 6 and lane 4 are compared, the amount of polyubiquitinated GST-OVA in lane 6 is significantly larger. This revealed that when HSP90 is present, the addition of KDEL (SEQ ID NO: 3) increases the uptake of GST-OVA and promotes polyubiquitination.

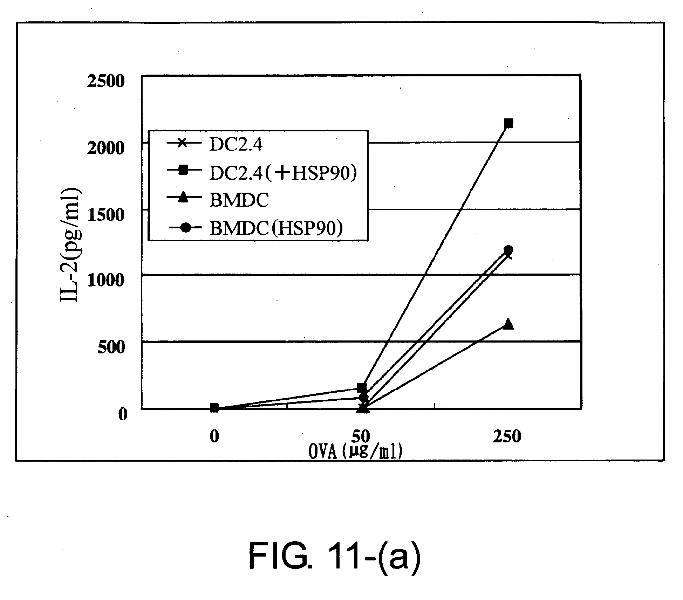

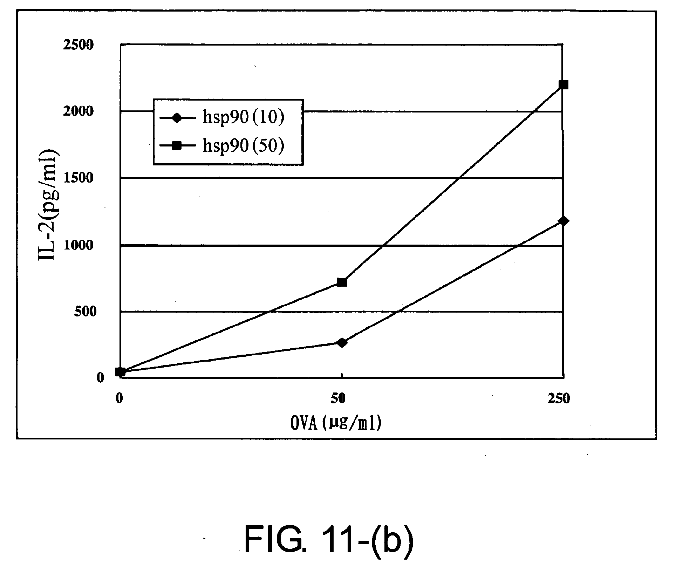

Fig. 11-(a) shows the results of incubating DC2.4 cells or BMDCs with OVA in the presence or absence of HSP90, and then measuring antigen presentation to OT-I CD8⁺ cells. Addition of HSP90 was shown to increase antigen presentation.
Fig. 11-(b) shows the results of measurements under the same conditions as for the data for DC2.4 cells in (a) above, but with different concentrations of Hsp90 (10 µg/mL and 50 µg/mL). This shows that antigen-presenting ability increases in a manner dependent on Hsp90 concentration.

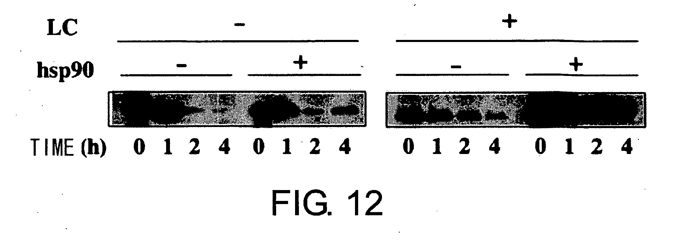

Fig. 12 shows the result of incubating DC2.4 cells with OVA in the presence or absence of lactacystin (LC), and in the presence or absence of Hsp90, and then measuring the OVA taken up into the cells. The time refers to incubation time. As the eight right-hand lanes show, uptake of OVA increased when Hsp90 was present.

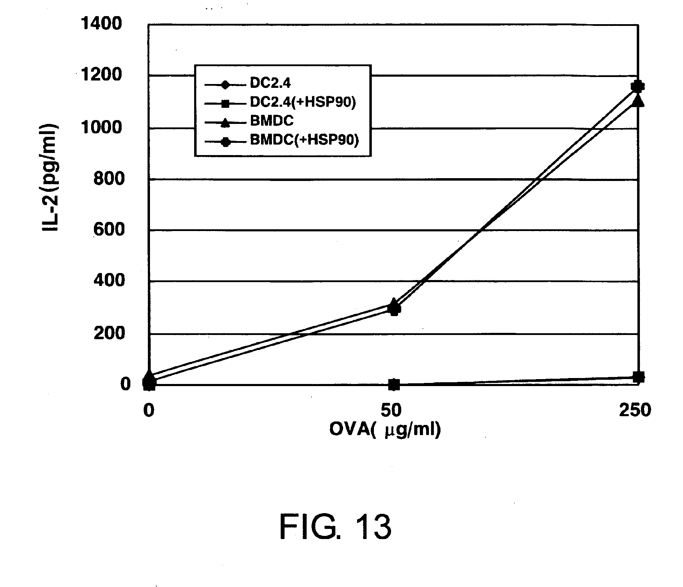

Fig. 13 shows the results of observing the effect of molecular chaperone HSP90 on foreign antigen presentation to MHC class II molecules on dendritic cells (DCs), measured in terms of the amount of IL-2 production.

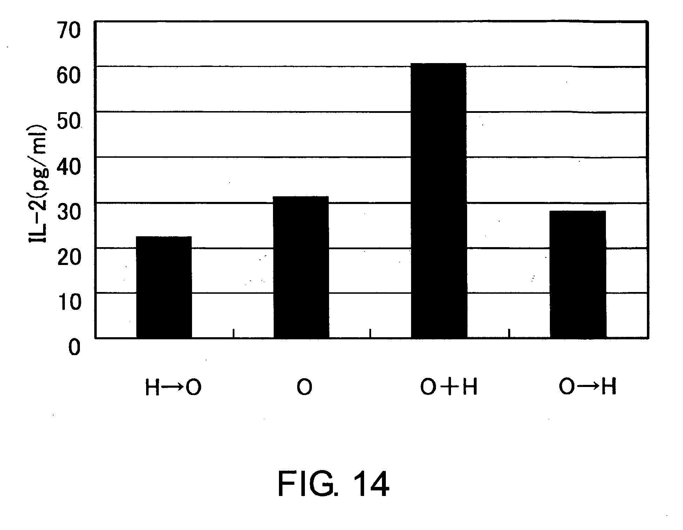

Fig. 14 shows the results of observing the change in the amount of IL-2 production by changing the timing of HSP90 and OVA addition to DC2.4 cells. In the figure, (H→O) refers to when HSP90 is first added to DC2.4 cells, followed by the addition of OVA. (O) refers to a control experiment in which only the OVA antigen is added, and HSP90 is not added. (O+H) refers to when OVA and HSP90 are simultaneously added to DC2.4 cells. (O→H) refers to when OVA is first added to DC2.4 cells, followed by the addition of HSP90.

### Best Mode for Carrying Out the Invention

The present invention relates to methods for producing dendritic cells that have acquired CTL-inducing ability, wherein the methods comprise the step of polyubiquitination promotion treatment that promotes the polyubiquitination of antigenic proteins in dendritic cells. In the present invention, dendritic cells that have acquired CTL-inducing ability refer to dendritic cells that present foreign antigenic peptides on MHC class I molecules on the cell surface. When CD8⁺ T cells recognize the antigen-derived peptides presented on MHC class I molecules on the surface of antigen-presenting cells, the CD8⁺ T cells are activated and CTLs exhibiting cytotoxicity are induced.

The present inventors revealed for the first time that in the process by which foreign antigenic proteins are degraded and the antigen-derived peptides are presented on MHC molecules, the antigenic proteins are polyubiquitinated in the dendritic cells prior to their degradation. In addition, various methods confirmed that when the aforementioned polyubiquitination in dendritic cells is promoted, CTL induction is promoted. Therefore, promotion of polyubiquitination in dendritic cells enables production of dendritic cells that are proficient at inducing CTLs.

Dendritic cells that present antigenic protein-derived peptides can be obtained by first contacting antigenic proteins with dendritic cells and culturing the cells. The production methods of the present invention comprise performing a treatment to promote polyubiquitination at the same time as or prior to contacting the dendritic cells with the antigenic proteins. The polyubiquitination promotion treatments enhance the level of antigen presentation on dendritic cells as compared to the case without treatment, and have the effect of further increasing CTL-inducing ability specific to the protein used as the antigenic protein.

Proteins used for the production methods of the present invention may be of any type or size so long as they can become subjects of phagocytosis by dendritic cells, and subjects of proteasome degradation. Generally, molecules composed of polypeptide chains are often differentiated by referring those with a molecular weight of 5000 or more as proteins, and those with a molecular weight of 5000 or less as peptides (Encyclopedic Dictionary of Chemistry, Tokyo Kagaku Dojin); however, so long as they are taken up by dendritic cells and undergo proteasome degradation, they may be used in the production methods of the present invention, without limitation on their molecular weight. Additionally, they may be simple proteins, or conjugated proteins such as nuclear proteins, lipoproteins, glycoproteins, chromoproteins, or metalloproteins. Cancer antigen proteins and infected virus-derived proteins can be suitably used in the present production methods.

The dendritic cells used in the methods of the present invention are preferably mammal-derived cells, and more preferably human-derived cells. The dendritic cells may be cells isolated from nature, or alternatively, artificially generated cell lines may be used. When selecting proteins for the purpose of performing tailor-made therapies, the use of cells derived from the patient to be treated is preferable.

The dendritic cells used in the methods of the present invention are preferably immature cells. This is because immature dendritic cells take up antigens from outside very well, and as they mature, they more strongly present the antigens that had been taken up. Such dendritic cells can be prepared by methods for using GM-CSF to induce them from CD34-positive progenitor cells in bone marrow or peripheral blood or from monocytes, or by methods for directly separating them from peripheral blood mononuclear cells, and other such methods.

Treatments to promote polyubiquitination in dendritic cells can be carried out by a number of methods. One example is a treatment that uses HSPs. The present inventors proved that HSP90 promotes polyubiquitination of antigenic proteins in dendritic cells and has the ability to increase the level of antigen presentation. HSP stands for heat shock protein, and was initially recognized as a protein whose expression is induced by heat shock stress. Later, HSPs were revealed to have the function of helping protein structure formation, and they came to be known as so-called 'molecular chaperones'. Molecular chaperones were originally defined as "proteins that chaperone the maturation of proteins in structurally immature states by temporarily binding to them". However, at present, molecular chaperones are known to be involved not only in protein folding, but also in the regulation of many cellular functions. HSPs are heat shock proteins; however, they are ubiquitously present even when the cells are not stressed.

HSPs constitute families comprising multiple members. The representative examples include the HSP60, HSP70, HSP90, and HSP100 families. The HSP90 family is one of the representative molecular chaperones, having a molecular weight of approximately 90 kDa. The members of this family are present not only in the cytosol but also in the endoplasmic reticulum and mitochondria. HSP90 is present in the cytosol of mammals. HSP90 may also be referred to as Hsp90 or hsp90. Two HSP90 isoforms are known to exist; in humans they are known as HSP90α and HSP90β, and in mice they may be called Hsp84 and Hsp86. In *Drosophila,* this protein is called hsp83; in budding yeast there are two types, Hsp82 and Hsc82; and in *Escherichia coli,* it is called HtpG. HSP90, in cooperation with other molecular chaperones or co-chaperones, has the function of suppressing aggregation of denatured proteins. Normally, it regulates the function of transcriptional regulators and molecules of the signal transduction system. GRP94 is a member that exists in the mammalian endoplasmic reticulum and is also referred to as gp96. Trap1/HSP75 is known to exist in mammalian mitochondria.

HSP70 family is a family of representative molecular chaperones that comprise an N-terminal ATPase domain having an acfin/hexokinase-type three dimensional structure and a C-terminal substrate-binding domain, and their molecular weights are approximately 70 kDa. The family has ATPase activity and promotes protein folding in cooperation with co-chaperones. It is also known to be involved in various aspects in the life span of a protein, such as in the transport of proteins to organelles and in repair/degradation of denatured proteins. Hsc70/Hsc73 are members constitutively present in the cytosol of mammals, and in mammals Hsp70/Hsp72 are inducible members known to be induced during stress such as heat shock. Grp78BiP are present in the endoplasmic reticulum of mammalian cells. DnaK is a member present in *E. coli.*

The HSP60 family is also called the chaperonins. The family member in *E. coli* is GroEL, and the member in the mitochondria of eukaryotic cells is called Hsp60. CCT/TRiC are present in the cytosol. All of these members form a double ring complex, and folding of substrate proteins proceeds in the central cavity of the ring.

HSP100 family members present in yeast are Hsp104 and Hsp78, and those present in mammals are Skd3 and ClpX.

Any of the various HSPs described above may be used in the production methods of the present invention, so long as they have a polyubiquitination promoting effect. HSPs belonging to the HSP90 or HSP70 family are preferably used, and HSP90 is more preferably used. The animal species from which the HSPs are derived may be the same as or different from the animal species from which the dendritic cells to be used are derived. As an example of HSP90, the amino acid sequence of porcine HSP90 is shown in SEQ ID NO: 1 and its cDNA sequence is shown in SEQ ID NO: 2. The sequence of human HSP90 can be obtained from GenBank Accession No. BC023006 and BC007989.

To promote polyubiquitination in dendritic cells using HSPs, methods of incubating HSPs with dendritic cells and antigenic proteins can be used, as described in the Examples. Various commercially available HSPs may be used. They may be obtained, for example, from Medical and Biological Laboratories Co., Stressgen Biotechnologies Corp., or Oxford Biomedical Research Inc. Alternatively, HSPs expressed in hosts, obtained by preparing HSP cDNAs from cDNA libraries of mammalian cells by using portions of the sequence of SEQ ID NO: 2 as primers and then inserting the obtained HSP cDNAs into appropriate expression vectors, can also be used.

Carrying out this operation promotes polyubiquitination of antigenic proteins in dendritic cells and increases antigen presentation by dendritic cells, and thus dendritic cells having the ability to induce CTLs can be produced. These methods can be carried out simply by contacting antigenic proteins with dendritic cells in the presence of HSPs, and can be said to be extremely convenient.

The promotion of polyubiquitination in dendritic cells can be confirmed by washing dendritic cells incubated with antigenic proteins and HSPs, producing an extract solution, and then detecting the polyubiquitinated antigenic proteins using anti-antigenic protein antibodies and anti-polyubiquitin antibodies.

Examples of different methods for promoting polyubiquitination in dendritic cells include methods of adding an endoplasmic reticulum localization sequence to the carboxyl terminus of antigenic proteins. The present inventors have confirmed the promotion of polyubiquitination and an increase in the level of antigen presentation when the endoplasmic reticulum localization sequence KDEL (SEQ ID NO: 3) is added to the C-terminus of antigenic proteins and these are contacted with dendritic cells. In the present invention, "endoplasmic reticulum localization sequence" refers to an amino acid sequence that has the function of directing shuttling (bidirectional transport) between the endoplasmic reticulum and Golgi apparatus. Representative examples of such sequences include the KDEL sequence (SEQ ID NO: 3) and the HDEL sequence (SEQ ID NO: 5). The KDEL sequence is a sequence comprising the amino acid sequence: Lys-Asp-Glu-Leu (SEQ ID NO: 3). Many of the soluble proteins localized in the endoplasmic reticulum have a KDEL sequence (SEQ ID NO: 3) on their C-terminus. Therefore, the KDEL sequence (SEQ ID NO: 3) has been thought to be a signal for remaining in the endoplasmic reticulum. However, KDEL receptors that bind to the KDEL sequence (SEQ ID NO: 3) were found to be present in the endoplasmic reticulum-Golgi intermediate compartment (ERGIC) and in the cis region of the Golgi apparatus. Currently, the KDEL sequence (SEQ ID NO: 3) is considered to be a signal for recovering, by retrograde transport, proteins that leaked from the endoplasmic reticulum. The HDEL sequence (SEQ ID NO: 5) is an endoplasmic reticulum localization sequence for budding yeasts and comprises the amino acid sequence His-Asp-Glu-Leu (SEQ ID NO: 5). In addition, there are reports that in ER-localized membrane proteins oriented such that their C-terminal side is on the cytoplasmic side, a C-tenninal DEKKMP sequence (amino acid sequence: Asp-Glu-Lys-Lys-Met-Pro; SEQ ID NO: 6) is a signal with similar function.

Antigenic proteins with an endoplasmic reticulum localization sequence added to their carboxyl terminus can be obtained by methods well known to those skilled in the art. For example, DNA sequences in which restriction enzyme recognition sequences have been added to both sides of a DNA encoding the KDEL sequence (SEQ ID NO: 3) are synthesized one strand at a time, these strands are annealed to form a double strand, and this is then trimmed using a suitable restriction enzyme. The C-terminal side of an antigenic protein is treated with the same restriction enzyme, an above DNA fragment comprising a DNA encoding the KDEL sequence (SEQ ID NO: 3) is ligated, and a DNA encoding an antigenic protein to which the KDEL sequence (SEQ ID NO: 3) has been added is obtained. The obtained DNA is inserted into a suitable expression vector, this is transfected into a host for expression, and the expressed recombinant protein is purified to obtain a desired protein. In another method, antigenic proteins with endoplasmic reticulum localization sequences added to their carboxyl terminus can be obtained by using PCR methods. For example, a primer complementary to the sequence encoding the N-terminal side of the antigenic protein is prepared as the forward primer, and a primer complementary to the sequence encoding the C-terminal side is prepared as the reverse primer. A DNA encoding an endoplasmic reticulum localization sequence is added to the 5' end of the reverse primer. When PCR methods are performed under suitable conditions using a reverse primer to which an endoplasmic reticulum localization sequence DNA has been added and an above forward primer, a DNA encoding an antigenic protein with an endoplasmic reticulum localization sequence added to its carboxyl terminus can be obtained. DNA obtained in this manner can be cloned, inserted into expression vectors, and then expressed in a host to obtain desired proteins. As an example of a DNA encoding an endoplasmic reticulum localization sequence that can be used in these methods, a DNA sequence encoding the KDEL sequence (SEQ ID NO: 3) is shown in SEQ ID NO: 4. The sequences are not limited to the sequence of SEQ ID NO: 4, so long as they are DNAs encoding the KDEL sequence (SEQ ID NO: 3), and other DNA sequences resulting from genetic code degeneracy may also be used. Similarly, any of the multiple DNA sequences that exist for other endoplasmic reticulum localization sequences may be used.

Appropriate expression vectors can be suitably selected in accordance with the translation system used for protein production. Cellular or cell-free translation systems can be selected, depending on the purpose. In cellular translation systems, vectors that can be used for expression in *Escherichia coli* include pKK223-3, pKK233-2, and pJLA502. The proteins can also be expressed as fusion proteins with other proteins. Examples of vectors for this purpose include pRIT2T, pGEX-2T, pGEX-3X, and such. These fusion proteins can be easily recovered using affinity columns. The use of a vector comprising a thrombin- or factor Xa-cleavage site at the junction of the fused proteins also enables specific recovery of the target protein. Examples of vectors for secretion of proteins into the periplasm or outside of bacteria include pKT280 and pRIT (Okada, M. and Miyazaki, K., ed. Formidable Biotechnical Series. Protein Experimental Note, Extraction and Separation/Purification, Yodosha, 1996, pp. 242-246). When producing the proteins using insect cells or mammalian cells, baculoviruses can be used. Examples of a baculovirus vector for mammalian cells include pAcCAGMCS1 (Muramatsu, M., ed. Laboratory Manual for Genetic Engineering, 3rd ed., Maruzen, 1996, pp. 242-246).

Recombinant proteins expressed in host cells can be purified by known methods. When proteins of the present invention are expressed in the form of fusion proteins linked at the N-terminus to a histidine residue tag, glutathione-S-transferase (GST), or such, the proteins can be purified through a nickel column, glutathione Sepharose column, or the like.

Alternatively, DNA sequences encoding amino acid sequences in which the KDEL sequence (SEQ ID NO: 3) is linked to an antigenic protein can also be chemically synthesized using nucleic acid synthesizers.

The production methods of the present invention can comprise a step of isolating dendritic cells derived from mammals before the above step of polyubiquitination promotion treatment. The types of mammals are not particularly limited, as described above. For example they may be pigs, cattle, horses, humans, monkeys, dogs, cats, mice, rats, or any other mammals. Methods for isolating dendritic cells can be carried out by known methods.

Furthermore, the production methods of the present invention may comprise a step of confirming the promotion of the polyubiquitination of antigenic proteins in dendritic cells, in addition to the above step of isolating dendritic cells and the above step of polyubiquitination promotion treatment. This collection step can be carried out, for example, as follows: a portion of dendritic cells subjected to the above step of a polyubiquitination promotion treatment is washed and an extract is produced, the amount of polyubiquitinated antigenic protein is measured using anti-antigenic protein antibodies and anti-polyubiquitin antibodies, and when this measured value is higher than the amount of the polyubiquitinated antigenic protein in dendritic cells not subjected to the step of polyubiquitination promotion treatment, the polyubiquitination of antigenic proteins can be judged to be promoted in these dendritic cells.

Dendritic cells produced by the present production methods are dendritic cells with a strong ability to induce CTLs specific to the protein used as the antigenic protein. By performing the present production methods using dendritic cells derived from cancer patients and by using a cancer antigen protein as the antigenic protein, patient-derived dendritic cells having an increased ability to induce CTLs that specifically impair the cancer antigen protein can be obtained. Such dendritic cells are thought to be valuable for use as dendritic cell vaccines with strong therapeutic effects against cancer. Further, by performing the present production methods using proteins derived from infectious microorganisms, dendritic cell vaccines for treating infections can be obtained.

The present invention also provides agents for promoting the polyubiquitination of antigenic proteins. The agents of the present invention for promoting polyubiquitination promote the polyubiquitination of antigenic proteins in dendritic cells.

One embodiment of the present invention is agents for promoting the polyubiquitination of antigenic proteins that include HSPs as active ingredients. As described above, HSPs can be used to promote the polyubiquitination of antigenic proteins in dendritic cells. Any of the various HSPs described above can be used as the HSPs to be used in the present promoting agents, so long as they have the effect of promoting polyubiquitination. Preferably members of the HSP90 family or HSP70 family are used, and more preferably HSP90 is used. The agents for promoting the polyubiquitination of antigenic proteins that include HSPs as active ingredients can be used by adding them when incubating the antigenic proteins and dendritic cells.

An example of another embodiment is agents that promote the polyubiquitination of antigenic proteins, which comprise DNAs encoding endoplasmic reticulum localization sequences. As described above, an endoplasmic reticulum localization sequence is an amino acid sequence that has the function of directing shuttling between the endoplasmic reticulum and the Golgi apparatus. For example, a DNA fragment encoding the KDEL sequence (SEQ ID NO: 3), which is a representative example of an endoplasmic reticulum localization sequence, can be included in a polyubiquitination-promoting agent of the present invention. By linking such DNA fragments downstream of DNAs encoding desired antigenic proteins, DNAs encoding antigenic proteins equipped with endoplasmic reticulum localization sequences are constructed. By synthesizing proteins from the DNAs constructed herein, antigenic proteins with endoplasmic reticulum localization sequences added to their C-terminal side can be obtained. The antigenic proteins with endoplasmic reticulum localization sequences added to their C-terminal side can be used to produce dendritic cells that have acquired the ability to induce CTLs, as described above.

DNAs encoding endoplasmic reticulum localization sequences may be included as is into the agents for promoting the polyubiquitination of antigenic proteins, or they may be included in a form carried by a vector. Vectors that harbor DNAs encoding endoplasmic reticulum localization sequences can be designed for operational convenience. For example, providing a multiple cloning site upstream of a DNA encoding an endoplasmic reticulum localization sequence will further facilitate the linking of the endoplasmic reticulum localization sequence to the C-terminal side of a desired antigenic protein.

The above agents for promoting the polyubiquitination of antigenic proteins can all be used to enhance the ability of dendritic cells to induce CTLs *via* the promotion of the polyubiquitination of antigenic proteins in dendritic cells.

All prior art references cited herein are incorporated by reference into this description.

### Examples

### [Example 1] Proteasome-dependent degradation of accumulated OVA

Intracellular localization of foreign antigens was examined by cell fractionation methods. Chicken ovalbumin (OVA) was used as an antigen model, and DC2.4 (haplotype H-2Kb), a cultured cell line of mouse dendritic cells, was used as a model for dendritic cells. The DC2.4 cell line is derived from dendritic cells, and was established while retaining many of the properties of dendritic cells, such as cross-presentation. BMDCs with a haplotype of H-2Kb, the same as that of DC2.4, were used as the control. BMDCs were induced from the bone marrow cells of C57BL/6 mice (SLC) in RPMI-1640 supplemented with 5% FCS using 5 ng/mL GM-CSF (MBL), and those cells purified by magnetic beads after five days of culturing were used.

DC2.4 cells made to take up bOVA antigens were suspended in 10 mM Tris-HCl (pH7.4) containing 250 mM sucrose, this was homogenized using glass beads, centrifuged for 30 minutes at 250,000 x g, and then fractionated. The supernatant was used as the soluble fraction; the precipitates were suspended in the same buffer as described above and used as the microsomal fraction. A fixed amount was incubated in the presence or absence of 1% TritonX-100, and together with or without 100 µg mL⁻¹ trypsin. Before collecting bOVA, soybean trypsin inhibitor (Sigma) was added at 500 µg mL⁻¹.

As a result of the above, 70% or more of bOVA taken up by cells was recovered as trypsin-resistant bOVA from the microsomal fraction. When bOVA collected in the microsomal fraction was treated with TritonX-100, it was degraded by trypsin. This suggests that most of the bOVA taken up was accumulated inside membranous subcellular structures (Fig. 1a). Further, BiP, an endogenous ER protein, was detected in the same microsomal fraction (Fig. 1a). Therefore, the microsomal fraction was found to comprise the ER fraction.

Next, DC2.4 cells were incubated with bOVA for one hour, and this was then chased for up to four hours. Cell-bound bOVA was recovered from total cell lysates by streptavidin-agarose and separated by SDS-PAGE. Cell-bound bOVA decreased with time, but this decrease was blocked in the presence of lactacystin or MG-132, which are proteasome inhibitors (Figs. 1b and 1c). Ammonium chloride, which is a lysosome inhibitor, did not significantly affect bOVA degradation (Fig. 1c). This suggests that lysosomal proteases are not involved in antigen degradation.

Essentially similar results were obtained from DC2.4 cells exposed to GST-OVA instead of bOVA and from bone marrow-derived DCs (BMDCs) exposed to bOVA (data not shown).

The majority of the cell-bound bOVA had the same molecular weight as untreated bOVA (Fig. 1d). In addition, a notable amount of bOVA was found to be polyubiquitinated, as shown by anti-polyubiquitin antibodies and anti-OVA antibodies (Fig. 1d). These results suggest that undegraded bOVA accumulates in membranous organelles and is degraded *via* the ubiquitin-proteasome pathway. Protein degradation with such characteristics is essentially the same as that known as ER-associated degradation (ERAD). The present inventors provide the following three lines of evidence indicating that bOVA accumulated in DC2.4 cells is degraded by ERAD, or if not identical, by a very similar protein degradation mechanism.

### [Example 2] Relationship between accumulated OVA and ERAD-related proteins

To prove the above-mentioned hypothesis, first, the relationship between accumulated OVA and substances involved in retrograde transport from the ER to the cytoplasm was examined by co-immunoprecipitation. 2 x 10⁷ DC2.4 or BMDC cells were cultured for four hours in a medium containing 10 µM MG-132 in the presence of bOVA at 250 µg mL⁻¹. Cells were lysed in 20 mM HEPES pH7.6 containing 1% digitonin and protease inhibitors to collect biotin-labeled samples. The samples were cleared of impurities in advance using protein G sepharose (Amersham Pharmacia Biotech), and after incubation with antibodies for precipitation (anti-Sec61β, anti Sec61α, anti-VCP, anti-BiP, anti-PDI, anti-calreticulin, anti-TAP1, and anti-TAP2), the immunoprecipitates were collected using protein G When chicken anti-CHIP antibody was used, immunoprecipitates were collected using a rabbit anti-IgY column (Mr. S. Seki, MBL). The precipitated samples were analyzed by SDS-PAGE and Western blotting.

When the Sec61 complex was immunoprecipitated with an anti-Sec61β antibody or anti-Sec61α antibody, a portion of bOVA was observed to be bound to Sec61 in DC2.4 cells incubated with bOVA in the presence or absence of lactacystin (Figs. 2a and 2b). Binding between bOVA and VCP was also indicated when a sample from DC2.4 cells incubated with bOVA was subjected to immunoprecipitation with an anti-VCP antibody (Fig. 2c). Three types of ER resident proteins - BiP, protein disulfide isomerase (PDI), and calreticulin - were also found to bind to OVA (Figs. 2d-f). Interestingly, a portion of bOVA was revealed to bind to CHIP, which is an E3-ubiquitin ligase (Fig. 2g). In contrast to the above-mentioned proteins, bOVA did not bind to TAP1 or TAP2 (Figs. 2h-j).

To further confirm the above binding between the ERAD-related proteins and bOVA, copurification experiments were carried out. Simultaneous purification experiments were carried out as follows: DC2.4 cells and BMDCs were individually incubated with bOVA (250 µg mL⁻¹), washed twice with PBS, and then lysed in a digitonin buffer (1% digitonin in 20 mM HEPES pH7.6 for use in copurification) together with a protease inhibitor cocktail (Sigma). The samples were precipitated using streptavidin-agarose beads (SA beads) (Novagen) for one hour at room temperature. The precipitated samples were separated on 7.5-15% SDS-PAGE (Biocraft) and transferred onto Immobilon P (Millipore) for Western blotting. The protein bands were visualized by chemiluminescence methods (Amersham Pharmacia Biotech).

Binding of BiP, Sec61α, Sec61β, VCP, calreticulin, and CHIP with bOVA was also demonstrated by copurification, from the cell lysates, of bOVA and the above proteins using streptavidin beads (Fig. 3a). The absence of binding between TAP1/2 and bOVA was again observed in the present purification experiments. Bone marrow-derived DC (BMDC) lysates incubated with anti-Sec61 antibody or anti-VCP antibody immunoprecipitated with bOVA (Figs. 2j and 2k), and BiP and VCP were co-purified with bOVA.

It is worth noting that the majority of bOVA, in DC2.4 cells, that bound to the above molecules including CHIP had the same molecular weight as untreated bOVA (Figs. 2a-g). These results suggest that denatured bOVA accumulates in the ER or ER-related structures without being degraded, is transported to the cytoplasm *via* Sec61 transporters, then undergoes polyubiquitination which at least partly involves CHIP, and is finally subjected to degradation by proteasomes.

### [Example 3] Decrease caused by ERAD inhibition of the degradation of accumulated OVA

Next, to further confirm the involvement of ERAD, the effect on bOVA accumulation of ERAD inhibition, for example by Ca²⁺ depletion or thapsigargin treatment, was examined. Depletion of Ca²⁺ in the ER is known to inhibit ERAD. Thapsigargin (Tg) is known to decrease the Ca²⁺ concentration in the ER lumen by binding to the ER membrane and functioning as an ionophore and to inhibit ERAD. Thus, in the presence of Tg, bOVA degradation by ERAD is predicted to be suppressed. DC2.4 cells were pulsed with bOVA in the presence of MG-132, then washed with PBS or with PBS containing 1 mM EGTA, and then chased for four hours. As shown in Fig. 4a, accumulated bOVA did not decrease with Ca²⁺ depletion. Re-addition of Ca²⁺ to cells pre-exposed to EGTA restored bOVA degradation (Fig. 4a). This eliminates the possibility that Ca²⁺ depletion caused irreversible damage to DC2.4 cells. Thapsigargin also inhibited the degradation of accumulated bOVA (Fig. 4b).

Furthermore, the effect of tunicamycin treatment on bOVA accumulation was examined. When cells are treated with tunicamycin (Tm), N-type glycosylation is inhibited, and abnormally folded proteins accumulate in the ER lumen. Since such structurally abnormal proteins are good ERAD substrates, treatment by ERAD of bOVA taken up is predicted to be competitively inhibited by the misfolded proteins formed by tunicamycin treatment. When DC2.4 cells were treated with tunicamycin, as expected, bOVA degradation was inhibited (Fig. 4b). Tunicamycin treatment resulted in the accumulation of N-glycosylation-deficient proteins in the ER, which were good ERAD substrates and therefore competed with bOVA for degradation.

As the third evidence for the above hypothesis, RNAi methods were performed to knock-down proteins involved in the retrograde transport of substrates in the ERAD pathway (Fig. 4c). A 1.2 kb *Pvu*II fragment derived from pSilencer1.0-U6 (Ambion) was inserted into the *Sma*I site of pEGFP-C1 (BD Biosciences) to obtain p60. p60ΔC was produced by deleting the 1.3 Kb *Ase*I-*Bgl*II fragment from p60. siRNA sequences targeting Sec61, VCP, or CHIP were inserted into p60ΔC (Sec61-22), p60 (VCP-41), or the pSilencer2.1-U6hygro vector (Ambion) (CHIP-4) according to the manufacturer's protocol. Out of five target sequences for Sec61α, two were significantly effective for Sec61α knock-down. Three were not effective. Two of the five target sequences for VCP and one of the five target sequences for CHIP were significantly effective. The effective target sequences used in the present Examples are the following:
Sec61-22: AATGATCATTACTATCGGT (SEQ ID NO: 7);
VCP-41: AATCCTTGAATGAAGTAGGCT (SEQ ID NO: 8); and
CHIP-4: CAGTATCGAGGAACGGCGC (SEQ ID NO: 9).

In Sec61α-knocked-down DC2.4 cells (S22 and S22-3), accumulated OVA decreased more slowly than in the parental DC2.4 cells (Figs. 4d and 4e). Scrambled siRNA sequences for Sec61α knock-down did not affect the expression level of Sec61α nor the accumulation of bOVA (data not shown). These results suggest that Sec61α is involved in the transport to direct bOVA to degradation. Similarly, VCP-knocked-down DC2.4 cells (V41 and V41-1) retained larger amounts of bOVA than parental DC2.4 cells during chase (Figs. 4d and 4e), although accumulated bOVA decreased to some extent.

When CHIP-knocked-down DC2.4 cells (C4 and C4F-1) were produced to examine the polyubiquitination of bOVA when CHIP is knocked down, the uptake of bOVA into the CHIP-knocked-down DC2.4 cells was only about 70% that of uptake into the parental DC2.4 cells (Figs. 4d and 4f). Despite this fact, polyubiquitination of bOVA was reduced to 34% in CHIP-knocked-down cells (Fig. 4f). The decrease in the amount of accumulated OVA was slowed down in CHIP-knocked-down cells (Figs. 4d and 4e). In contrast, TAP1 knock-down did not affect bOVA degradation in DC2.4 cells (Fig. 5).

### [Example 4] Localization of accumulated OVA in DC2.4 cells

All of the above results strongly support that bOVA taken up by DC2.4 cells is degraded through ERAD. However, proteomic analyses and immunoelectron microscopic observations have recently revealed that phagosomes, which are where infectious bacteria are taken up, comprise a group of proteins that constitute the ER, including TAP1/2 and substances involved in antigen presentation, such as MHC I. Accordingly, the present inventors decided to examine the intracellular localization of accumulated OVA in DC2.4 cells and BMDCs by using immunofluorescence confocal microscopy. DC2.4 cells and BMDCs on culture slides (FALCON) were pretreated for 30 minutes with MG-132, and then incubated for two hours with OVA at 500 µg mL⁻¹. After washing three times with the medium, the cells were fixed for ten minutes with 3.7% formaldehyde, permeabilized by treating with 0.1% TritonX-100 in PBS for ten minutes at room temperature, incubated with a designated antibody in PBS containing 5% BSA/2% FCS at 37°C for one hour, and stained. Alexa Fluor 488 goat anti-mouse IgG F(ab')₂ fragments, Alexa Fluor 488 goat anti-rat IgG, and Alexa Fluor 546 goat anti-rabbit F(ab')2 fragments (Molecular Probes) were used as the secondary antibodies. Images were captured using an 1X70 (Olympus) inverted microscope equipped with a fluorescence apparatus and processed using Deltavision deconvolution microscopy software (Applied Precision).

Spots or speckles of OVA were detected in the cells, but these OVA distributions did not overlap with either early endosomes (immunostaining of EEA1) or caveosomes (immunostaining of caveolin-1), which are known to be involved in the uptake of foreign substances (Figs. 6a and 6b). Instead, OVA immunofluorescence colocalized at least in part with late endosomes (stained with anti-Lamp1 antibody) (Fig. 6c) and ER (Fig. 6d). Although ER labeled with the anti-KDEL antibody showed a lace-like structure, OVA was not uniformly distributed throughout the ER (Fig. 6d). Similarly, OVA was detected in restricted areas of late endosomes (Fig. 6c). OVA did not colocalize with the Golgi apparatus (stained with anti-GM-130 antibody) (Fig. 6f). Localization of accumulated OVA in the ER was also confirmed with BMDCs (Fig. 6e). These results are consistent with the hypothesis of the present inventors that the OVA taken up into DC follows ERAD and/or a similar protein degradation pathway in other organelles comprising ER constituents, such as phagosomes and late endosomes.

### [Example 5] Subcellular fractionation of OVA-containing membrane vesicles by density-gradient centrifugation

Next, the present inventors examined the distribution of accumulated OVA in membrane fractions of DC2.4 cells by using iodixanol gradient centrifugation. For the density gradient centrifugation, DC2.4 cells incubated with bOVA (500 µg/mL) for three hours were washed twice with PBS, suspended in a homogenization medium (0.25 M sucrose, 1 mM EDTA, and 10 mM Hepes-NaOH pH7.4), and ruptured by ten strokes of a Dounce homogenizer. Unruptured cells and nuclei were removed by centrifugation at 2,000 x g for ten minutes. The nuclei-removed (postnuclear) supernatant was centrifuged at 100,000 x g for 45 minutes to obtain a pellet, this was resuspended in a homogenization medium and layered onto a 2.5-30% or 10-30% discontinuous Optiprep (Gibco/Invitrogen) gradient. Centrifugation was carried out at 4°C with a Beckman SW 60Ti rotor, at 200,000 x g for 2.5 hours in the case of 2.5-30% gradient, and at 300,000 x g for three hours in the case of 10-30% gradient Eleven fractions obtained from the top layer of each centrifuge tube were TCA precipitated, and then analyzed by SDS-PAGE and Western blotting.

Although OVA was detected in all the fractions, the peak of OVA was clearly observed in fraction #10 in the 2.5-30% density gradient (Fig. 7a) and in fraction #5 in the 10-30% density gradient (Fig. 7b). Distribution of Sec61, BiP (KDEL), and Rab5 in the fractions was similar to that of OVA (Fig. 7). In contrast, Lamp-1, a late endosome marker, showed two peaks-fractions #6 and #10 - in 2.5-30% density gradient. GM130, a Golgi marker, and transferrin receptor (Tfr), a recycling endosome marker, were collected separately. All of these results are consistent with those obtained by immunofluorescence microscopy.

### [Example 6] Reduction of cross-presentation due to knock-down of Sec61, VCP and CHIP

Whether knock-down of cellular components involved in ERAD decreases the cross-presentation of exogenous OVA antigen was examined using a system for evaluating the level of antigen presentation, which uses naive CD8⁺ T cells from OT-I mice and DC2.4 cells. The DC2.4 cells used as a model of dendritic cells are of the H-2Kb mouse MHC class I haplotype. OT-I (H-2Kb) mice are transgenic mice expressing a T cell receptor (TCRα and β) that recognizes an antigenic peptide (SIINFEKL) (SEQ ID NO: 15) derived from OVA presented on the H-2Kb molecules. The CD8⁺ cells of OT-I mice are activated when they recognize OVA peptides presented by BMDCs derived from H-2Kb mice or by DC2.4 cells. When OVA or bOVA is incubated with and affixed onto DC2.4 cells and the cells are further incubated with naive CD8⁺ T cells of OT-I mice, the amount of IL-2 produced by OT-I CD8⁺ T cells serves as an index of T-cell stimulation as a result of OVA antigen presented by DC2.4 cells.

Parental or knocked-down DC2.4 cells were pre-incubated in a culture medium supplemented with 50 µg mL⁻¹ polymyxin B for 30 minutes in the presence or absence of lactacystin or MG-132, and incubated with OVA (250 µg mL⁻¹) or SIINFEKL peptides (10 ng mL⁻¹) for six hours. The cells were washed, fixed with 1% paraformaldehyde, and aliquoted into microtiter plates (5 x 10⁴ per well). 5 x 10⁴ CD8⁺ T cells purified from splenocytes of OT-1 mice using magnetic beads were added to each well and after a 24-hour incubation, the IL-2 secretion was measured by ELISA (BD Biosciences) in triplicate.

As shown in Fig. 8a, OVA and bOVA were equally antigenic for stimulation of CD8+ OT-I T cells. As expected, proteasome inhibitors suppressed OVA antigen presentation (Fig. 8b). In contrast, NH₄Cl did not reduce the presentation of the OVA antigen with MHC class I to OT-I CD8⁺ T cells, but inhibited the presentation with MHC class II to OT-II CD4⁺ cells (Fig. 9). The effect of knocking down Sec61, VCP, or CHIP was examined using uncloned DC2.4 transfectants into which siRNA expression vectors had been introduced (S22, V41, and C4), as well as cloned transfectants (S22-3, V41-1, and C4-1) (Figs. 8c and 8d). Since Sec61 is involved in both forward and retrograde transport of ER-associated proteins, it was not surprising that the knock-down of Sec61 reduced antigen presentation of OVA added from outside the cell. Knock-down of VCP necessary for the retrograde transport of ER-associated proteins also reduced antigen presentation by DC2.4 cells. These results support the hypothesis of the present inventors that exogenously added OVA is processed for cross-presentation *via* ERAD. Furthermore, in accordance with the effect of OVA accumulation in DC2.4 cells, knock-down of CHIP resulted in a decrease in OVA cross-presentation (Figs. 8c and 8d), suggesting that CHIP functions as an E3-ligase for OVA polyubiquitination during cross-presentation.

### [Example 7] Promotion of polyubiquitination by addition of an endoplasmic reticulum localization sequence

There are reports that when the KDEL sequence (Lys-Asp-Glu-Leu) (SEQ ID NO: 3), an endoplasmic reticulum localization sequence, is added to the carboxyl terminus of a protein localized in the ER lumen, the protein is repeatedly transported between the ER and Golgi apparatus, and ERAD of that protein is promoted. Therefore, DC2.4 cells were made to take up OVA with KDEL (SEQ ID NO: 3) added to its C-terminus, and OVA that was polyubiquitinated after uptake was observed by SDS-PAGE and Western blotting. OVA to which a KDEL sequence (SEQ ID NO: 3) was added was constructed and expressed as follows: using a plasmid comprising a DNA sequence encoding OVA as a template, PCR was performed using oligo-1 (SEQ ID NO: 10) and oligo-2 (SEQ ID NO: 11) as primers. The amplified PCR product of approximately 1100 bp was introduced into a pCR-Blunt vector (Invitrogen) to obtain vector-1. Oligo-3 (SEQ ID NO: 12) and oligo-4 (SEQ ID NO: 13) were annealed, and the obtained gene fragment of approximately 100 bp was introduced into the pCR-Blunt vector to obtain vector-2. Vector-2 was digested with *Spe*I*,* the obtained gene fragment of approximately 130 bp was introduced into vector-1 that had been digested with *Nhe*I to obtain vector-3. An approximately 1250-bp gene fragment (OVA-KDEL) obtained when vector-3 was digested with *BamH*I/*Spe*I was introduced into a vector for gene expression, and then expressed. The amino acid sequence of OVA to which a KDEL sequence (SEQ ID NO: 3) has been added is shown in SEQ ID NO: 14.

The results are shown in Fig. 10. Compared to when DC2.4 cells were made to take up OVA, uptake of OVA which has a KDEL (SEQ ID NO: 3) added to its C-terminus resulted in significantly promoted polyubiquitination of the substrate (OVA-KDEL).

Moreover, (Lys-Asp-Glu-Leu) (SEQ ID NO: 3) was added to the carboxyl terminus of OVA, DC2.4 cells were made to take this up, and the antigen presentation levels of OVA and OVA-KDEL were observed using the aforementioned antigen presentation level evaluation system that uses OT-I mouse CD8⁺ T cells. Antigen presentation was clearly found to be stronger with OVA-KDEL than with OVA (data not shown).

The above results showed that when OVA and OVA-KDEL are compared, OVA-KDEL is more readily polyubiquitinated, and more efficiently presented as antigen. Therefore, a correlation was found between ease of polyubiquitination and ease of presentation as an antigen. Therefore, the degree of cross-presentation by an antigenic protein can be estimated from the results of measuring the polyubiquitination of the antigenic protein.

When OVA is the external antigen, as described above, an antigen presentation assay using OT-I mouse cells can be constructed. However, in general, animals that express TCR specific to a particular antigen do not exist, therefore, construction of an antigen presentation assay such as that with OVA is impossible. Therefore, when there is a need to know how easily a certain antigen is presented, it is very useful to estimate the ease of antigen presentation by measuring the uptake of the antigenic protein into dendritic cells and the polyubiquitination of the antigenic protein.

### [Example 8] Promotion of polyubiquitination by Hsp

DC2.4 cells were incubated with OVA (0, 50, or 250 µg/mL) in RPMI1640/10% FCS for six hours at 37°C, in the presence or absence of pig-derived HSP90 (10 µg/mL). After washing away the antigen and HSP, OT-I CD8⁺ T cells were added, cells were cultured overnight at 37°C, and the amount of IL-2 comprised in the supernatant was measured by the ELISA method. As a result, a stronger antigen presentation was observed than when incubation was carried out with OVA alone (Fig. 11-(a)). Moreover, the antigen presentation ability increased in an Hsp90 concentration-dependent manner (Fig. 11-(b)).

Next, DC2.4 cells were incubated for a set amount of time with OVA in the presence or absence of lactacystin (LC), and OVA taken up into the cells was measured. OVA taken up into cells in the presence or absence of Hsp90 was measured similarly. DC2.4 cells were observed to take up OVA into cells more efficiently in the presence of Hsp90 than in the absence of Hsp90 (Fig. 12).

DC2.4 cells were incubated with GST-OVA in the presence or absence of Hsp90, cells were collected, and an extract solution was produced. After subjecting this to SDS-PAGE, Western blotting was performed using an anti-polyubiquitin antibody. OVA taken up into DC2.4 cells were shown to be more strongly polyubiquitinated in the presence of Hsp90 than in the absence of Hsp90 (Fig. 10).

According to the above results, compared to incubation with OVA alone, incubation with OVA in the presence of Hsp90 increases the amount of OVA taken up by cells, and the amount of polyubiquitination was also found to increase. It can be concluded from this finding that measuring the antigen presentation of a certain antigenic protein can be substituted with measuring the uptake of an antigenic protein into dendritic cells and the polyubiquitination of the antigenic protein.

### [Example 9] Effects of the molecular chaperone HSP90 on foreign antigen presentation on MHC class II of dendritic cells (DC)

Mouse bone marrow-derived dendritic cells (BMDCs) were prepared as follows: cells were taken out from mouse bone marrow and cultured for five days in RPMI medium containing GM-CSF and 5% FCS, during which the medium was partially exchange. This culturing method leads to growth of macrophages, dendritic cells, and such; therefore, dendritic cells alone were collected using CD11c magnetic beads, and these cells were used as bone marrow-derived dendritic cells (BMDCs).

The bone marrow-derived dendritic cells (BMDCs) prepared as above or the mouse dendritic cell line DC2.4 cells were plated in a 96-well plate at 5 x 10⁴ cells per well. Next, in the presence of 50 µg/mL of polymyxin B, which is an endotoxin inhibitor, OVA (0, 50, or 250 µg/mL) and HSP90 (0 or 10 µg/mL) were added, the total fluid volume was adjusted to 200 µL, and this was cultured overnight at 37°C. Since endotoxin is known to activate DCs, polymyxin B was added to eliminate the effects of its contamination of reagents, equipment, and such. After culturing, the aforementioned cells in the wells were washed twice to remove the remaining OVA and HSP90 that were not taken up by the cells. CD4⁺ T cells prepared from OT-II transgenic mice were added to this at 5 x 10⁴ cells per well, the total fluid volume was adjusted to 200 µL, and this was cultured overnight. The wells were centrifuged, the supernatant was collected, and the amount of IL-2 in the supernatant was measured by ELISA.

The results are shown in Fig. 13. BMDCs took up OVA, activated CD4⁺T cells derived from OT-II, and were shown to induce IL-2 production. These activations are dependent on the concentration of OVA. However, in contrast to activation of OT-I mouse-derived CD8⁺ T cells (Fig. 11-(a)), IL-2 production (DC activation) was not enhanced even when 10 µg/mL of HSP90 was added to the cells at the same time as OVA. On the other hand, DC2.4 cells used in this experiment have a low level of MHC class II molecule expression and did not activate OT-II-derived CD4⁺ T cells at a significant level.

OT-I transgenic mice carry T cell receptors that recognize the states in which ovalbumin-derived peptides are presented on MHC class I molecules. Reaction between peptide-MHC class I on cells and T cell receptors on CD8⁺ T cells stimulates T cells to produce IL-2. Meanwhile, OT-II transgenic mice carry T cell receptors that can recognize the states in which ovalbumin-derived peptides are presented on MHC class II molecules. Reaction between peptide-MHC class II on antigen presenting cells and T cell receptors on CD4⁺ T cells stimulates T cells to produce IL-2. From the results obtained when using OT-I mouse-derived CD8⁺ T cells (Fig. 11-(a)) and the results obtained when using OT-II-derived CD4⁺ T cells (Fig. 13), it is clear that the molecular chaperone HSP90 enhances presentation of the foreign antigen OVA on MHC class I molecules by dendritic cells (DCs) to CD8⁺ T cells, but does not enhance presentation on MHC class II molecules to CD4⁺ T cells. In other words, HSP90 enhances the activation of cytotoxic T cells (CTLs) by DCs, but does not enhance the activation of helper T cells.

### [Example 10] Examination of the effect of the timing of HSP addition

The timing of the addition of HSP90 (10 µg/mL) and OVA (250 µg/mL) to DC2.4 cells was varied, and the effect on class I antigen presentation was observed. (i) HSP90 was added first, DC2.4 cells were washed, and then OVA was added. (ii) HSP90 and OVA were added simultaneously to DC2.4 cells. (iii) OVA was added first, and after washing DC2.4 cells, HSP90 was added. The results obtained under these three conditions were compared. As a control for all these conditions, an experiment in which only OVA, but not HSP90, was added was also carried out.

The procedures for the experiments carried out under these three conditions are specifically described below:

Under condition (i), HSP90 was added to DC2.4 cells, the cells were cultured for 30 minutes and then washed to remove HSP90. Next, OVA was added and the cells were cultured for three hours. Thereafter, OVA was removed by washing, and cells were cultured in just the culture medium for three hours. DC2.4 cells activated with OVA by the above procedure were plated in a 96-well plate, OT-I mouse-derived CD8⁺ T cells were added to this, and after culturing overnight, the amount of IL-2 released into the supernatant was measured by ELISA.

Under condition (ii), OVA and HSP90 were added simultaneously to DC2.4 cells, cells were cultured for three hours, then washed, and cultured in just the culture medium for three hours. Thereafter, the procedures to investigate the activation of OT-I mouse-derived CD8⁺ T cells was carried out similarly to in (i) above.

Under condition (iii), OVA was first added to DC2.4 cells, cells were cultured for three hours, and then washed to remove OVA. Next, HSP90 was added, and the cells were cultured for another three hours. The procedures that follow were carried out similarly to that of (i) and (ii). Meanwhile, timings were set such that the incubation times during the stage in which cells are in contact with OVA and the stage at which OVA has been taken up were the same under conditions (i) to (iii).

The results are shown in Fig. 14. Columns (H→O), (O), (O+H), and (O→H) indicate the results from condition (i), the control, condition (ii), and condition (iii), respectively. An increase in the amount of IL-2 production as compared to the control was observed only under condition (ii), in which OVA and HSP90 were added simultaneously to DC2.4 cells. These results indicate that the effect of HSP90 cannot be observed unless it is added simultaneously with the OVA antigen.

It was revealed that the effect of HSP90 on class I antigen presentation of OVA by DC2.4 cells is not exhibited unless HSP90 coexists with OVA. Moreover, HSP90 was shown not to yield any effect on OVA that has already been taken up (Fig. 14, column (O→H)). HSP90 is predicted to enhance the uptake of the OVA antigen or its processing. Further, this result agrees well with the results that HSP90 somewhat enhances the uptake of OVA and that it significantly enhances the polyubiquitination of the OVA taken up (Fig. 10).

### Industrial Applicability

The present invention has provided new methods for producing dendritic cells that have acquired CTL-inducing ability. The production methods of the present invention are methods that enable the production of dendritic cells in which antigen cross-presentation is enhanced. Since the dendritic cells produced by the production methods of the present invention are effective for inducing CTL activation, it is thought they may be used effectively for immunotherapy in cancer treatment.

## Claims

1. A method for producing a dendritic cell that has acquired CTL-inducing ability, wherein the method comprises the step of a polyubiquitination promotion treatment that promotes the polyubiquitination of an antigenic protein in a dendritic cell.

2. A method for producing a dendritic cell that has acquired CTL-inducing ability, wherein the method comprises the steps of:
(a) isolating a mammal-derived dendritic cell; and
(b) a polyubiquitination promotion treatment that promotes the polyubiquitination of an antigenic protein in the dendritic cell.

3. A method for producing a dendritic cell that has acquired CTL-inducing ability, wherein the method comprises the steps of:
(a) isolating a mammal-derived dendritic cell;
(b) a polyubiquitination promotion treatment that promotes the polyubiquitination of an antigenic protein in the dendritic cell; and
(c) confirming the promotion of polyubiquitination of an antigenic protein in the dendritic cell.

4. The method of any one of claims 1 to 3 for producing a dendritic cell that has acquired CTL-inducing ability, wherein the step of a polyubiquitination promotion treatment is a step that comprises contacting an antigenic protein and an HSP with an isolated dendritic cell.

5. The production method of claim 4, wherein the HSP is an HSP belonging to the HSP90 family or HSP70 family.

6. The production method of claim 5, wherein the HSP belonging to the HSP90 family is HSP90.

7. The method of any one of claims 1 to 3 for producing a dendritic cell that has acquired CTL-inducing ability, wherein the step of a polyubiquitination promotion treatment is a step that comprises contacting an antigenic protein, in which an endoplasmic reticulum localization sequence has been added to its carboxyl terminus, with an isolated dendritic cell.

8. The production method of claim 7, wherein the endoplasmic reticulum localization sequence is KDEL (SEQ ID NO: 3).

9. An agent for promoting the polyubiquitination of an antigenic protein, which comprises an HSP as an active ingredient.

10. The agent of claim 9 for promoting the polyubiquitination of an antigenic protein, in which the HSP is HSP90.

11. An agent for promoting the polyubiquitination of an antigenic protein, which comprises a DNA encoding an endoplasmic reticulum localization sequence.

12. An agent for promoting the polyubiquitination of an antigenic protein, which comprises a vector harboring a DNA encoding an endoplasmic reticulum localization sequence.

13. An agent for enhancing the CTL-inducing ability of a dendritic cell, which comprises the agent of any one of claims 9 to 12 for promoting the polyubiquitination of an antigenic protein, as an active ingredient.
